# EUROPÄISCHE PATENTSCHRIFT

(11) **EP 3 033 373 B1**
(45) Veröffentlichungstag und Bekanntmachung des Hinweises auf die Patenterteilung: **17.10.2018**
(21) Anmeldenummer: 14742184.6
(22) Anmeldetag: 16.07.2014
(51) Int. Cl.: C08G 63/91, C07C 67/08

(54) **CARBOXYFUNKTIONELLE POLY- UND DIESTERDERIVATE**
CARBOXYFUNCTIONAL POLY AND DIESTER DERIVATIVES
DÉRIVÉS DE POLY- ET DIESTERS À FONCTION CARBOXY

(30) Priorität: 16.08.2013 EP 13180646
(43) Veröffentlichungstag der Anmeldung: 22.06.2016
(73) Patentinhaber: BASF Coatings GmbH, 48165 Münster (DE)
(72) Erfinder: HINTZE-BRÜNING, Horst, 48165 Münster (DE); AUSTRUP, Elke, 59394 Nordkirchen (DE); LOHMEIER, Thomas, 32369 Rahden (DE); FAUL, Charles Frederick James, Bristol BS6 6XN (GB); OLIVER, Alex Matthew, Lemington Spa Warwickshire CV31 1TR (GB)
(74) Vertreter: BASF IP Association
(86) Internationale Anmeldenummer: PCT/EP2014/065260
(87) Internationale Veröffentlichungsnummer: WO 2015/022134

(56) Entgegenhaltungen:
- WO-A1-99/11693
- WO-A1-2010/130312
- GB-A- 1 526 290
- US-A1- 2005 176 881

## Beschreibung

Die vorliegende Erfindung betrifft neue carboxyfunktionelle Poly- und Diesterderivate, die herstellbar sind durch ringöffnende Umsetzung eines Anhydrids mit einer hydroxyfunktionellen Vorstufe. Während die hydroxyfunktionelle Vorstufe einen hydrophoben Charakter aufweist, entstehen durch die ringöffnende Umsetzung freie Carboxylgruppen und demzufolge ein amphiphiler Molekülcharakter des resultierenden Poly- oder Diesterderivats. Die erfindungsgemäßen Poly- und Diesterderivate weisen hervorragende anwendungstechnische Eigenschaften auf. Insbesondere ist es möglich, mit den erfindungsgemäßen Poly- und Diesterderivaten wässrige Zusammensetzungen herzustellen, welche flüssigkristalline Eigenschaften aufweisen. Das heißt also, dass Zusammensetzungen, welche die erfindungsgemäßen Poly- und Diesterderivate sowie Wasser enthalten, flüssigkristalline Phasen ausbilden.

### Stand der Technik

Unterschiedlichste Polyester sowie Diesterderivate und ihre Verwendung sind bekannt. Insbesondere Polyester werden beispielsweise als Filmbildner in Beschichtungsmitteln wie Lacken und Farben eingesetzt. Ebenfalls eingesetzt werden solche Ester beispielsweise, je nach Zusammensetzung und Eigenschaften, als Additive zur Erzielung unterschiedlichster Eigenschaften in Beschichtungsmitteln wie Lacken und Farben. Dabei ist es auch möglich, dass solche Poly- und Diesterderivate ihre Eigenschaften durch spezifische Kombination mit weiteren Komponenten wie Additiven, Pigmenten und/oder Füllstoffen entfalten.

So offenbart WO 2010/130312 A1 den Einsatz einer spezifischen Kombination eines speziellen Polyesters mit schichtförmigen anorganischen Teilchen, beispielsweise gemischten Hydroxiden, in wässrigen Systemen, wobei diese Systeme flüssigkristalline Eigenschaften aufweisen. Diese flüssigkristallinen Systeme führen bei Verwendung in wässrigen Beschichtungsmitteln zu einer hervorragenden Steinschlagfestigkeit von mit diesen Beschichtungsmitteln hergestellten Beschichtungen.

WO 2010/130308 offenbart ebenfalls eine Kombination aus Polyester und gemischten Hydroxiden beziehungsweise Hydrotalciten in wässriger Phase und den Einsatz dieser flüssigkristallinen Systeme in wässrigen Effektwasserbasislacken. Die optischen Eigenschaften, insbesondere die Erzielung von Metallic-Effekten (Flop) und Helligkeit von mit den Effektlacken hergestellten Lackierungen sind hervorragend.

Flüssigkristalline Systeme sind solche, die neben typischen Eigenschaften einer Flüssigkeit, insbesondere einer gewissen Fließfähigkeit beziehungsweise Fluidität auch typische Eigenschaften eines Kristalls, insbesondere also eine gewisse strukturelle Anisotropie, aufweisen. Solche Systeme können beispielsweise thermotrop sein, das heißt ihre flüssigkristallinen Eigenschaften treten temperaturabhängig auf. Ebenfalls findet man lyotrope Systeme. In solchen Systemen wird bei Anwesenheit eines Lösungsmittels, beispielsweise Wasser, und amphiphilen organischen Komponenten wie Tensiden innerhalb gewisser Konzentrationsbereiche eine Flüssigkristallinität erreicht. Bei vorhandener struktureller Anisotropie ist regelmäßig auch eine optische Anisotropie gegeben, das heißt solche Systeme zeigen das bekannte Phänomen der Doppelbrechung. Die Systeme weisen also für unterschiedliche Polarisation und Richtung des eingestrahlten Lichts unterschiedliche Brechungsindices auf. Die Doppelbrechung führt dazu, dass unter dem Polarisationsmikroskop (das heißt gekreuzten Polarisatoren, nämlich dem Primärfilter beziehungsweise dem Linearpolarisator des Lichtes der Lichtquelle sowie dem hierzu um 90° gedrehten Sekundärfilter beziehungsweise Analysator) trotz der genannten Filteranordnung und anders als bei rein isotropen Systemen keine vollständige Auslöschung auftritt, sondern ein Teil des Lichtes durch den Analysator dringt und damit detektiert werden kann.

Die Einflüsse der Flüssigkristallinität und der damit vorhandenen Balance zwischen Anisotropie und Fließfähigkeit auf unterschiedliche Eigenschaften wie beispielsweise Pigmentorientierung und/oder Rheologie, macht die entsprechenden Systeme besonders nutzbringend. Zum einen können die durch den flüssigen Charakter bestehenden Vorteile, insbesondere die gute Formulierbarkeit und Verarbeitbarkeit, genutzt werden. Zum anderen gelingt durch die Kristallinität und die entsprechende Anisotropie die gleichzeitige Nutzung der entsprechenden Eigenschaften von festen Komponenten. Es besteht somit ein Bedarf an der Bereitstellung neuer Komponenten beziehungsweise Verbindungen, mit denen die Erzielung der genannten Flüssigkristallinität möglich ist. Insbesondere in wässrigen Systemen, die gerade im Bereich der Lackindustrie aufgrund ihres wertvollen ökologischen Profils von hoher Relevanz sind, ist die Bereitstellung solcher Komponenten von großem Interesse.

### Aufgabe

Aufgabe der vorliegenden Erfindung war demzufolge die Bereitstellung neuer Komponenten, mit denen Systeme mit besonders ausgeprägten flüssigkristallinen Eigenschaften hergestellt werden können. Insbesondere sollten Komponenten bereitgestellt werden, die in wässrigen Systemen zu diesen flüssigkristallinen Eigenschaften führen können. Dabei sollten die Systeme möglichst einfach hergestellt werden können und das Erreichen einer Flüssigkristallinität auch ohne aufwendigen Zusatz weiterer Verbindungen ermöglicht werden können. Das heißt also, die Komponenten sollten für sich allein und ohne den Zusatz weiterer Verbindungen in beziehungsweise mit Wasser zu flüssigkristallinen Phasen führen. Auch wenn sich in unterschiedlichen Fällen die Kombination mit weiteren Verbindungen anbieten kann, sollte somit die Möglichkeit eröffnet werden, die Komponenten für sich allein in Wasser einzusetzen und dabei flüssigkristalline Phasen auszubilden. Auf diese Weise sollte es gelingen, die anisotropen, geordneten Eigenschaften von kristallinen Systemen mit der einer Flüssigkeit beziehungsweise einem fließfähigen System eigenen guten Formulierbarkeit zu vereinen.

### Lösung

Es wurde gefunden, dass die genannten Aufgaben gelöst werden konnten durch neue carboxyfunktionelle Poly- und Diesterderivate, die herstellbar sind durch ringöffnende Umsetzung von
(A) mindestens einem Anhydrid der Formel (I): 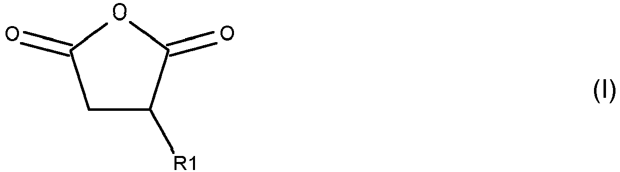 wobei R₁ = H, C₁- bis C₄₈-Alkyl, C₂- bis C₄₈-Alkenyl,
   mit
(B) mindestens einer hydroxyfunktionellen Komponente, wobei
(B1) zur Herstellung der Polyesterderivate mindestens ein linearer Polyester, der zwei endständige Hydroxylgruppen aufweist, bei dessen Herstellung 7 bis 95 mol-%, bezogen auf die Gesamtmenge der bei der Herstellung des Polyesters (B1) eingesetzten Monomere, mindestens ein difunktionelles Monomer (b1) mit aliphatischen Gruppen mit 12 bis 70 Kohlenstoffatomen zwischen den funktionellen Gruppen eingesetzt wird,
   und
(B2) zur Herstellung der Diesterderivate mindestens eine dihydroxyfunktionelle Komponente, welche eine aliphatische Gruppe mit 12 bis 70 Kohlenstoffatomen zwischen den Hydroxylgruppen aufweist,
   eingesetzt wird.

Die neuen carboxyfunktionellen Poly- und Diesterderivate werden im Folgenden auch als erfindungsgemäße Poly- und Diesterderivate bezeichnet. Bevorzugte Ausführungsformen der erfindungsgemäßen Poly- und Diesterderivate sind der weiter unten folgenden Beschreibung sowie den Unteransprüchen zu entnehmen.

Weiterhin Gegenstand der vorliegenden Erfindung ist ein Verfahren zur Herstellung der erfindungsgemäßen Poly- und Diesterderivate bei dem
(A) mindestens ein Anhydrid der Formel (I) 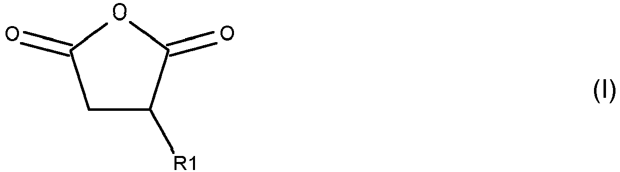 wobei R₁ = H, C₁- bis C₄₈-Alkyl, C₂- bis C₄₈-Alkenyl
   mit
(B) mindestens einer hydroxyfunktionellen Komponente unter Öffnung des Anhydridrings umgesetzt wird, wobei
(B1) zur Herstellung der Polyesterderivate mindestens ein linearer Polyester, der zwei endständige Hydroxylgruppen aufweist, bei dessen Herstellung 7 bis 95 mol-%, bezogen auf die Gesamtmenge der bei der Herstellung des Polyesters (B1) eingesetzten Monomere, mindestens ein difunktionelles Monomer (b1) mit aliphatischen Gruppen mit 12 bis 70 Kohlenstoffatomen zwischen den funktionellen Gruppen eingesetzt wird,
   und
(B2) zur Herstellung der Diesterderivate mindestens eine dihydroxyfunktionelle Komponente, welche eine aliphatische Gruppe mit 12 bis 70 Kohlenstoffatomen zwischen den Hydroxylgruppen aufweist,
   eingesetzt wird.

Zudem Gegenstand der vorliegenden Erfindung ist eine wässrige Zusammensetzung enthaltend mindestens ein erfindungsgemäßes Poly- und/oder Diesterderivat sowie Wasser.

Ebenfalls Gegenstand der vorliegenden Erfindung ist ein Verfahren zur Herstellung von flüssigkristallinen Phasen unter Einsatz mindestens eines erfindungsgemäßen Poly- und/oder Diesterderivats sowie die Verwendung der erfindungsgemäßen Poly- und Diesterderivate zur Herstellung flüssigkristalliner Phasen, insbesondere die Verwendung zur Herstellung solcher Phasen mit Wasser.

Insbesondere wurde gefunden, dass mit den erfindungsgemäßen Poly- und Diesterderivaten eine besonders ausgeprägte Flüssigkristallinität in wässrigen Systemen erzielt werden kann. Dabei können diese wässrigen Systeme auf sehr einfache Weise hergestellt werden. Auch kann die Flüssigkristallinität ohne den Zusatz weiterer Verbindungen erreicht werden, sodass der Zusatz weiterer Verbindungen nur dann notwendig ist, wenn dies im Einzelfall gewünscht ist, beispielsweise zum Erreichen verschiedener weiterer anwendungstechnischer Eigenschaften. Die Systeme vereinbaren also die anisotropen, geordneten Eigenschaften von kristallinen Systemen mit der einer Flüssigkeit beziehungsweise einem fließfähigen System eigenen guten Formulierbarkeit.

### Ausführliche Beschreibung

### Anhydrid (A)

Zur Herstellung der erfindungsgemäßen Poly- und Diesterderivate wird mindestens ein Anhydrid (A) der folgenden Formel (I) eingesetzt: 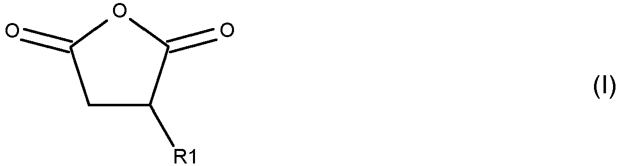 wobei R₁ = H, C₁- bis C₄₈-Alkyl, C₂- bis C₄₈-Alkenyl, bevorzugt C₆- bis C₄₈-Alkyl, C₆-bis C₄₈-Alkenyl, besonders bevorzugt C₆- bis C₄₈-Alkenyl und ganz besonders bevorzugt C₆- bis C₂₄-Alkenyl.

Bei dem Anhydrid (A) handelt es sich also um Bernsteinsäureanhydrid oder um entsprechend alkyl- beziehungsweise alkenylsubstituierte Bernsteinsäureanhydridderivate, bevorzugt alkenylsubstituierte Bernsteinsäurederivate. Die Alkyl- und Alkylenreste können linear oder verzweigt sein. Dabei kann die Kohlenstoff-Kohlenstoff-Doppelbindung in den Alkylenresten an sich beliebig angeordnet sein. Bevorzugt werden die Bernsteinsäureanhydridderivate durch Reaktion von Maleinsäureanhydrid mit alpha-Olefinen hergestellt. Dabei entsteht zunächst ein entsprechendes alkenylmodifiziertes Bernsteinsäureanhydridderivat, welches dann auch im Sinne der vorliegenden Erfindung eingesetzt werden kann. Ein solches Derivat enthält dann vom Anhydridring aus betrachtet in beta-gamma-Position eine Kohlenstoff-Kohlenstoff-Doppelbindung. Beispielhaft genannt sei 2-Octenylbernsteinsäureanhydrid, welches im Rahmen der vorliegenden Erfindung zudem ganz besonders bevorzugt eingesetzt wird. Der zugrundeliegende Reaktionsmechanismus (En-Reaktion) ist dem Fachmann bekannt, entsprechende Reaktionsbedingungen können problemlos ausgewählt und angepasst werden. Entsprechende Verbindungen können auch kommerziell erworben werden.

Möglich ist beispielsweise auch, dass ein entsprechendes Bernsteinsäurederivat, beispielsweise 2-Octenylbernsteinsäureanhydrid, mit weiteren in der Reaktionslösung vorhandenen alpha-Olefinen, beispielsweise 1-Octen, über eine En-Reaktion weiterreagiert und auf diese Weise durch Anknüpfung des weiteren Moleküls des alpha-Olefins eine Verzweigung in die Alkenylkette eingebaut wird. Die dabei entstehenden Mischungen von Bernsteinsäureanhydridderivaten können ebenfalls kommerziell erworben werden und ebenfalls zur Herstellung der erfindungsgemäßen Poly- und Diesterderivate eingesetzt werden. Bevorzugt werden aber molekular einheitliche Derivate eingesetzt. Solche einheitlichen Derivate können, wie bereits oben angegeben, auch kommerziell erworben werden.

### Komponente (B)

Die zur ringöffnenden Umsetzung eingesetzte Komponente (B) ist hydroxyfunktionell. Auf diese Weise gelingt die weiter unten im Detail beschriebene ringöffnende Umsetzung mit dem Anhydrid unter Bildung von Esterbindungen und freien Carboxylgruppen.

Zur Herstellung der Polyesterderivate werden als Komponente (B) lineare, hydroxyfunktionelle Polyester (B1) eingesetzt.

Als Polyester wird in der Regel eine polymere organische Verbindung bezeichnet, die unter Einsatz mehrwertiger organischer Alkohole und mehrwertiger organischer Carbonsäuren hergestellt wird. Die Alkohole und Carbonsäuren werden dabei durch Veresterung, das heißt also durch Kondensationsreaktionen, miteinander verknüpft. Entsprechend werden die Polyester in der Regel der Gruppe der Polykondensationsharze zugeordnet. Zur Herstellung von Polyestern können bekanntermaßen auch statt oder neben den entsprechenden organischen Carbonsäuren die Anhydride der Carbonsäuren, insbesondere die Anhydride von Dicarbonsäuren, eingesetzt werden. Im Rahmen der vorliegenden Erfindung ist unter der Bezeichnung Anhydrid also ein Carbonsäureanhydrid zu verstehen. Ebenfalls möglich ist die Herstellung durch den Einsatz von Hydroxycarbonsäuren oder den von den Hydroxycarbonsäuren durch intramolekulare Veresterung abgeleiteten Lactonen.

Lineare Produkte wie die erfindungsgemäß einzusetzenden Polyester (B1) werden insbesondere beim Einsatz von difunktionellen Ausgangskomponenten (Diole, Dicarbonsäuren) erhalten. Der Ausdruck linearer Polyester bedeutet im Rahmen der vorliegenden Erfindung also, dass das Polymerrückgrat, das heißt also die Abfolge von Esterbindungen, welche die Verknüpfung der einzelnen Polyesterbausteine ausmachen, linearen Charakter hat. Die einzelnen zur Herstellung eingesetzten Verbindungen (Monomere) besitzen also jeweils zwei zur Esterbindung befähigte funktionelle Gruppen, das heißt also insbesondere Hydroxylgruppen, Carboxylgruppen, und/oder Anhydridgruppen. Durch die Verknüpfung entsteht dann eine Polyesterkette beziehungsweise ein linearer Polyester. Bevorzugt werden dementsprechend nur untergeordnete Mengen (weniger als 5 mol-%, bevorzugt weniger als 2 mol-%, bezogen auf die Gesamtmenge der bei der Herstellung des Polyesters (B1) eingesetzten Monomere) von Monomeren eingesetzt, die mehr als zwei zur Esterbindung befähigte funktionelle Gruppen aufweisen, da hierdurch Verzweigungen in die Polymermoleküle eingefügt werden können. Ganz besonders bevorzugt werden keine Monomere eingesetzt, die mehr als zwei zur Esterbindung befähigte funktionelle Gruppen aufweisen.

Als Monomere, die zur Herstellung der Polyester (B1) eingesetzt werden, werden im Rahmen der vorliegenden Erfindung alle bei der Herstellung dieser Polyester (B1) eingesetzten einzelnen Ausgangsverbindungen, deren Grundgerüste in den Polyester eingebaut werden, bezeichnet. Dabei handelt es sich beispielsweise um typische monomere Verbindungen mit zwei entsprechenden funktionellen Gruppen wie beispielsweise 1,6-Hexandiol. Als Ausgangsverbindungen zur Herstellung von Polyestern können bekanntermaßen aber auch Verbindungen eingesetzt werden, die selbst bereits durch auf unterschiedlichste Weise mögliche Verknüpfung mehrerer einzelner Moleküle hergestellt sind. Beispielhaft sei auf die unten beschriebenen dimeren Fettsäuren verwiesen. Jedoch sind auch diese Verbindungen als Ausgangsverbindungen zu bezeichnen, die durch entsprechende Polymerisationsreaktionen in den Polyester eingebaut werden und dann einen nicht selbstständiger Anteil am Polyester ausmachen. Demzufolge werden auch diese Ausgangsverbindungen als Monomere bezeichnet.

Die zur Herstellung der erfindungsgemäßen Polyesterderivate eingesetzten Polyester (B1) sind solche, bei deren Herstellung 7 bis 95 mol.-%, bezogen auf die bei der Herstellung des Polyesters (B1) eingesetzten Monomere, mindestens ein difunktionelles Monomer (b1) mit aliphatischen Gruppen mit 12 bis 70 Kohlenstoffatomen zwischen den funktionellen Gruppen eingesetzt wird.

Aliphatische Verbindungen sind bekanntermaßen acyclische oder cyclische, gesättigte oder ungesättigte Kohlenstoffwasserstoffverbindungen, die nicht aromatisch sind. Der Begriff aliphatische Verbindung umfasst also acyclische und cyclische Aliphaten und gilt auch im Rahmen der vorliegenden Erfindung als entsprechender Oberbegriff. Die acyclischen Aliphaten können linear oder verzweigt sein. Linear bedeutet in diesem Zusammenhang bekanntermaßen, dass die jeweilige Verbindung keine Verzweigungen hinsichtlich der Kohlenstoffkette aufweist, sondern die Kohlenstoffatome ausschließlich in linearer Abfolge in einer Kette angeordnet sind. Verzweigt beziehungsweise nicht-linear bedeutet damit im Rahmen der vorliegenden Erfindung, dass die jeweils betrachtete Verbindung eine Verzweigung in der Kohlenstoffkette aufweist, das heißt also anders als bei den linearen Verbindungen mindestens ein Kohlenstoffatom der jeweiligen Verbindung ein tertiäres oder quartäres Kohlenstoffatom ist. Als cyclische Aliphaten beziehungsweise Cycloaliphaten werden solche Verbindungen bezeichnet, in denen zumindest ein Teil der vorhandenen Kohlenstoffatome im Molekül so verknüpft sind, dass einer oder mehrere Ringe ausgebildet werden. Natürlich können neben dem einen oder den mehreren Ringen weitere acyclische lineare oder verzweigte aliphatische Gruppen in einem Cycloaliphaten vorhanden sein.

Eine aliphatische Gruppe ist demzufolge eine Gruppe, die die oben für die aliphatischen Verbindungen genannten Voraussetzungen erfüllt, jedoch nur ein Teil eines Moleküls ist. Neben der aliphatischen Gruppe sind in dem entsprechenden Molekül noch andere Gruppen wie beispielsweise funktionelle Gruppen enthalten. Als funktionelle Gruppen werden im Rahmen der vorliegenden Erfindung endständige Gruppen bezeichnet, die Heteroatome wie Sauerstoff, Schwefel und/oder Stickstoff enthalten, beispielsweise Hydroxylgruppen oder Carboxylgruppen. Enthalten sein können selbstverständlich auch verbrückende Heteroatome beziehungsweise verbrückende Gruppen enthaltend Heteroatome. Beispielhaft seien Etherbindungen genannt.

Als Monomere mit aliphatischen Gruppen zwischen funktionellen Gruppen werden folglich solche Monomere bezeichnet, die neben den entsprechenden funktionellen Gruppen aliphatische Gruppen, welche zwischen den funktionellen Gruppen angeordnet sind, aufweisen. Die Monomere bestehen also aus entsprechenden funktionellen Gruppen und aliphatischen Gruppen, enthalten also lediglich die (endständigen) funktionellen Gruppen und die aliphatischen Gruppen.

Die funktionellen Gruppen der difunktionellen Monomere (b1) sind augenscheinlich solche, die zur Bildung von Esterbindungen befähigt sind, das heißt also insbesondere Hydroxylgruppen und/oder Carbonsäuregruppen sowie Anhydridgruppen. Die Monomere (b1) sind also bevorzugt Diole, Dicarbonsäuren und/oder Hydroxycarbonsäuren sowie Anhydride, insbesondere bevorzugt Diole und/oder Dicarbonsäuren.

Die aliphatischen Gruppen der Monomere (b1) besitzen 12 bis 70, bevorzugt 13 bis 50 und besonders bevorzugt 14 bis 40 Kohlenstoffatome.

Zur Herstellung der Polyester (B1) werden, bezogen auf die Gesamtmenge der bei der Herstellung des Polyesters (B1) eingesetzte Monomere, 7 bis 95 mol-%, bevorzugt 9 bis 90 mol-%, besonders bevorzugt 10 bis 85 mol-% der Monomere (b1) eingesetzt.

In einer ganz besonders bevorzugten Ausführungsform der vorliegenden Erfindung werden zur Herstellung der Polyester (B1), bezogen auf die Gesamtmenge der bei der Herstellung des Polyesters (B1) eingesetzte Monomere, 50 bis 95 mol-%, bevorzugt 60 bis 90 mol-%, besonders bevorzugt 70 bis 85 mol-% der Monomere (b1) eingesetzt.

Bevorzugte Monomere (b1) sind beispielsweise vollständig hydrierte Bisphenole, beispielsweise das vollständig hydrierte Bisphenol A. Genauso bevorzugt sind dimere aliphatische Fettalkohole und/oder dimere aliphatische Fettsäuren, wobei darunter die dimeren aliphatischen Fettsäuren bevorzugt sind. In einer weiteren bevorzugten Ausführungsform werden sowohl dimere aliphatische Fettsäuren und dimere aliphatische Fettalkohole gemeinsam als Monomere (b1) eingesetzt.

In einer weiteren besonders bevorzugten Ausführungsform werden im Rahmen der vorliegenden Erfindung dimere aliphatische Fettsäuren mit 24 bis 40 Kohlenstoffatomen sowie vollständig hydriertes Bisphenol A als Monomere (b1) eingesetzt.

Dimere aliphatische Fettsäuren sind herstellbar durch katalytische Dimerisierung von pflanzlichen, ungesättigten Fettsäuren, wobei insbesondere die 18 Kohlenstoffatome enthaltenden ungesättigten Fettsäuren zur Herstellung eingesetzt werden, das Produkt also 36 Kohlenstoffatome aufweist. Die Verknüpfung verläuft vornehmlich nach dem Diels-Alder-Typ und der En-Reaktion und es resultieren Gemische aus beispielsweise cycloaliphatischen und linear-aliphatischen dimeren Fettsäuren, welche je nach Mechanismus und/oder gegebenenfalls nachträglicher Hydrierung gesättigt oder ungesättigt sein können. Neben den aliphatischen Addukten (b1) enthalten diese Gemische meist auch gewisse Anteile an aromatischen beziehungsweise gemischt aliphatisch-aromatischen Gruppen.

Demzufolge werden, sofern als Monomere (b1) auch dimere aliphatische Fettsäuren eingesetzt werden, neben diesen Monomeren (b1) zur Herstellung der Polyester (B1) bevorzugt auch difunktionelle Monomere (b2) eingesetzt, die ebenfalls 12 bis 70 Kohlenstoffatome, bevorzugt 13 bis 50 und besonders bevorzugt 14 bis 40 Kohlenstoffatome zwischen den funktionellen Gruppen enthalten, die aber nicht rein aliphatisch sind, sondern zumindest anteilig auch aromatisch sind. Bevorzugt sind diese Gruppen also gemischt aliphatisch-aromatisch, das heißt die Gruppen mit 12 bis 70 Kohlenstoffatomen enthalten aliphatische und aromatische Teile.

Die genannten dimeren Fettsäuren beziehungsweise Monomere (b1) können in entsprechenden Gemischen mit Monomeren (b2) als Handelsprodukte erhalten werden. Zu nennen sind beispielsweise die dimeren aliphatischen Fettsäuren der Pripol®-Serie der Fa. Unichema.

Das molare Verhältnis der dimeren aliphatischen Fettsäuren (b1) zu den Monomeren (b2) in diesen Handelsprodukten ist bevorzugt von 2 bis 6, insbesondere bevorzugt 3 bis 5. Denn solche Verhältnisse resultieren regelmäßig bei der oben beschriebenen katalytischen Dimerisierung von Fettsäuren.

Es werden demnach in einer bevorzugten Ausführungsform Gemische von als Monomere (b1) einzusetzende dimere aliphatische Fettsäuren und Monomeren (b2) eingesetzt. Bevorzugt ist das molare Verhältnis dieser Gemische von 2 bis 6, insbesondere bevorzugt von 3 bis 5.

Die Monomere (b2) werden also, je nach gewünschter Menge von als Monomere (b1) einzusetzenden dimeren aliphatischen Fettsäuren, bezogen auf die Gesamtmenge der bei der Herstellung des Polyesters (B1) eingesetzte Monomere, bei der Herstellung des Polyesters (B1) in Anteilen von beispielsweise 1 bis 10 mol-%, bevorzugt 2 bis 5 mol-%, eingesetzt. Nach oben Gesagtem gilt, dass der Anteil der Monomere (b2) davon abhängig ist, ob und in welchem Anteil als Monomere (b1) dimere Fettsäuren und damit die genannten Handelsprodukte eingesetzt werden. Werden als Monomere (b1) beispielsweise ausschließlich oder überwiegend hydrierte Bisphenole und/oder dimere aliphatische Fettalkohole (die regelmäßig durch Hydrierung von dimeren aliphatischen Fettsäuren erhalten werden, wobei dann die regelmäßig auch vorhandenen Monomere (b2) ebenfalls hydriert werden und damit zu Monomeren (b1), nämlich dimeren aliphatischen Fettalkoholen, reduziert werden) eingesetzt, so ist der Anteil der Monomere (b2) entsprechend geringer. Werden ausschließlich oder überwiegend dimere aliphatische Fettsäuren eingesetzt, so sind die Anteile der Monomere (b2) somit offensichtlich in der Regel höher.

Als weitere Bausteine können bei der Herstellung des Polyesters (B1) bevorzugt die folgenden Monomere eingesetzt werden:
- (b3) lineare aliphatische und/oder cycloaliphatische Diole mit 2 bis 11 Kohlenstoffatomen, wie insbesondere Ethylenglykol, Diethylenglykol, 1,3-Propandiol, Dipropylenglykol, 1,4-Butandiol, 1,6-Hexandiol, 1,4-Cyclohexandiol und/oder 1,4-Dimethylolcyclohexan, bevorzugt 1,4-Butandiol und/oder 1,6-Hexandiol, wobei diese Diole bei der Herstellung des Polyesters (B1) bevorzugt in Anteilen von beispielsweise 0 bis 40 mol-%, bevorzugt 0 bis 35 mol-%, bezogen auf die Gesamtmenge der bei der Herstellung des Polyesters (B1) eingesetzten Monomere, eingesetzt werden,
- (b4) verzweigte aliphatische Diole mit 4 bis 11 Kohlenstoffatomen, wie insbesondere Neopentylglykol, 2-Methyl-2-propylpropandiol, 2-Ethyl-2-butylpropandiol, 2,2,4-Trimethyl-1,5-pentandiol, 2,2,5-Trimethyl-1,6-hexandiol, bevorzugt Neopentylglykol, wobei diese Diole bei der Herstellung des Polyesters (B1) bevorzugt in Anteilen von beispielsweise 0 bis 45 mol-%, bevorzugt 0 bis 40 mol-%, bezogen auf die Gesamtmenge der bei der Herstellung des Polyesters (B1) eingesetzten Monomere, eingesetzt werden,
- (b5) lineare aliphatische, cycloaliphatische und/oder aromatische Dicarbonsäuren mit 4 bis 12 Kohlenstoffatomen, wie insbesondere Oxalsäure, Malonsäure, Bernsteinsäure, Glutarsäure, Adipinsäure, Sebacinsäure, Maleinsäure, Fumarsäure, Isophthalsäure, Terephthalsäure, Orthophthalsäure, Tetrahydrophthalsäure, Hexahydrophthalsäure, 1,4-Cyclohexandisäure beziehungsweise deren Anhydride, bevorzugt Hexahydrophthalsäure, wobei diese Dicarbonsäuren bei der Herstellung des Polyesters (B1) bevorzugt in Anteilen von beispielsweise 5 bis 30 mol-%, bevorzugt 10 bis 25 mol-%, bezogen auf die Gesamtmenge der bei der Herstellung des Polyesters (B1) eingesetzten Monomere, eingesetzt werden,
- gegebenenfalls weitere Monomere (b6) mit beispielsweise 12 bis 70 Kohlenstoffatomen, welche verbrückende Heteroatome beziehungsweise verbrückende Gruppen enthaltend Heteroatome enthalten, in Anteilen von beispielsweise 0 bis 10 mol-%, bezogen auf die Gesamtmenge der bei der Herstellung des Polyesters (B1) eingesetzten Monomere.

Bevorzugt werden die zur Herstellung der Polyester (B1) eingesetzten Monomere in solchen Anteilen eingesetzt, dass das molare Verhältnis von Hydroxylgruppen zu Carbonsäuregruppen bei größer 1,5, insbesondere zwischen 1,5 und 3, ganz besonders bevorzugt zwischen 1,7 und 2,7 und in einer ganz bevorzugten Ausführungsform zwischen 1,8 und 2,5 liegt. In die Berechnung dieses molaren Verhältnisses gehen die zwei potentiellen Carbonsäuregruppen eines Anhydridmonomers auf Seiten der Carbonsäuregruppen ein. Durch die Wahl dieser bevorzugten Verhältnisse gelingt es besonders gut, die Hydroxyfunktionalität der Polyester (B1) zu generieren, insbesondere zwei endständige Hydroxylgruppen zur nachfolgenden ringöffnenden Umsetzung mit dem Anhydrid (A) zu generieren. Systeme beziehungsweise Polyesterderivate, die unter Einsatz solcher Polyester (B1) hergestellt werden und bei denen der Polyester (B1) beidseitig Anhydridfunktionalisiert wird, weisen besonders gute Eigenschaften hinsichtlich der Flüssigkristallinität auf und sind demnach besonders bevorzugt. Die angegebenen molaren Verhältnisse verstehen sich als aus der jeweiligen Funktionalität und dem Molekulargewicht der eingesetzten Ausgangsverbindungen (Monomere) errechnete Verhältnisse.

Der Polyester (B1) ist hydroxyfunktionell. Erfindungsgemäß ist, dass er pro Molekül genau zwei endständige Hydroxylgruppen aufweist, das heißt also die lineare Polyesterkette wird beidseitig durch eine Hydroxylgruppe abgeschlossen.

Der Polyester (B1) weist bevorzugt eine OH-Zahl von 80 bis 200, besonders bevorzugt von 100 bis 190, ganz besonders bevorzugt von 120 bis 180 mg KOH/g auf. Die OH-Zahl wird im Rahmen der vorliegenden Erfindung gemäß DIN 53240 bestimmt. Die Säurezahl des Polyesters (B1) ist bevorzugt niedrig und liegt besonders bevorzugt im Bereich von 0 bis 50, bevorzugt 2 bis 30, ganz besonders bevorzugt von 5 bis 20 mg KOH/g. Bevorzugt enthält der Polyester (B1) also einen nur sehr geringen Anteil an Carboxylgruppen, beispielsweise lediglich rein synthesebedingt restliche Carboxylgruppen, die durch einen nicht ganz vollständigen Umsatz zurückbleiben. Die Säurezahl wird im Rahmen der vorliegenden Erfindung gemäß DIN EN ISO 3682 gemessen. Im Rahmen der vorliegenden Erfindung versteht sich die Angabe einer OH-Zahl oder Säurezahl eines Polymers immer in Bezug auf den entsprechenden nichtflüchtigen Anteil. Der nichtflüchtige Anteil (Festkörper) einer Komponente, beispielsweise einer Dispersion eines Polymers wie einem Polyester, wird im Rahmen der vorliegenden Erfindung nach DIN EN ISO 3251 mit einer Einwaage von 1,0 g der jeweiligen Komponente bei einer Prüfdauer von 60 min und bei einer Temperatur von 125 °C bestimmt.

Das zahlenmittlere Molekulargewicht des Polyesters (B1) liegt bevorzugt im Bereich von 400 bis 2000, insbesondere bevorzugt von 600 bis 1500 g/mol, ganz besonders bevorzugt 700 bis 1200 g/mol, während das gewichtsmittlere Molekulargewicht bevorzugt im Bereich von 1200 bis 3000 g/mol, insbesondere bevorzugt 1500 bis 2500 g/mol liegt. Die Bestimmung der Molekulargewichte erfolgt im Rahmen der vorliegenden Erfindung mittels GPC-Analyse mit THF (+0,1% Essigsäure) als Eluent (1 ml/min) auf einer Styrol-Divinylbenzol-Säulenkombination. Die Kalibrierung wird mit Polystyrol-Standards durchgeführt.

Die Herstellung der Polyester (B1) und damit die Umsetzung der Monomere erfolgt nach den allgemein gut bekannten Methoden der Polyesterchemie, wobei der Fachmann ebenfalls weiß, wie er die Bedingungen zu wählen hat, um beispielsweise die oben genannten bevorzugten Eigenschaften, beispielsweise OH-Zahl und Säurezahl, zu erhalten. Die Umsetzung kann beispielsweise in Masse oder in Lösung mit typischen organischen Lösemitteln bei Temperaturen von beispielsweise 50°C bis 300°C, bevorzugt 100°C bis 290°C, insbesondere 140°C bis 280°C erfolgen. Insbesondere durch Reaktionstemperaturen von über 140°C kann gewährleistet werden, dass bei gleichzeitigem Einsatz von Anhydriden und freien Carbonsäuren auch eine effektive Umsetzung von freien Carbonsäuren, das heißt ein effektiver Einbau der entsprechenden Monomere in das Polyestergerüst, von statten geht. Selbstverständlich können auch typische Katalysatoren wie Schwefelsäure, Sulfonsäuren und/oder Tetraalkyltitanate, Zink- beziehungsweise Zinnalkoxylate, Dialkylzinnoxide oder organische Salze der Dialkylzinnoxide zum Einsatz kommen. Zur Herstellung der Diesterderivate werden als Komponente (B) dihydroxyfunktionelle Komponenten (B2) eingesetzt, wobei eine Komponente (B2) eine aliphatische Gruppe mit 12 bis 70 Kohlenstoffatomen zwischen den Hydroxylgruppen aufweist. Für die Komponente (B2) gilt hinsichtlich der aliphatischen Gruppe und der entsprechenden Definitionen das bereits oben hinsichtlich des Monomers (b1) beschriebene. Die Komponente (B2) besteht also aus einer aliphatischen Gruppe mit 12 bis 70 Kohlenstoffatomen und den Hydroxylgruppen. Bevorzugt weist die Komponente (B2) 13 bis 50 und besonders bevorzugt 14 bis 40 Kohlenstoffatome auf. Bevorzugt werden als Komponente (B2) dihydroxyfunktionelle Komponenten eingesetzt, in denen die Hydroxylgruppen sekundär sind. Es hat sich gezeigt, dass sich hierdurch insbesondere die Hydrolysestabilität der erfindungsgemäßen Diesterderivate in wässrigem Medium verbessern lässt, das heißt also insbesondere die Hydrolysestabilität der Esterbindung, die durch die weiter unten beschriebene ringöffnende Umsetzung der Hydroxylgruppen von Komponente (B2) mit dem Anhydrid (A) entsteht.

Bevorzugte Komponenten (B2) sind damit beispielsweise die vollständig hydrierten Bisphenole. Ganz besonders bevorzugt ist das vollständig hydrierte Bisphenol A.

In bestimmten Ausführungsformen der vorliegenden Erfindung kann aber auch der Einsatz von dimeren aliphatischen Fettalkoholen oder eine Kombination von vollständig hydrierten Bisphenolen, insbesondere vollständig hydriertem Bisphenol A, und dimeren aliphatischen Fettalkoholen von Vorteil sein.

### Die ringöffnende Umsetzung zur Herstellung der erfindungsgemäßen Poly- und Diesterderivate

Die Herstellung der erfindungsgemäßen Poly- und Diesterderivate erfolgt durch ringöffnende Umsetzung eines Anhydrids (A) der weiter oben gezeigten und erläuterten Formel (I) mit einer wie oben beschriebenen hydroxyfunktionellen Komponente (B).

Wie der Fachmann weiß, erfolgt eine solche Umsetzung zwischen einem Anhydrid und einer Hydroxylgruppe unter Bildung einer Esterbindung und einer Carboxylgruppe. Die hydroxyfunktionelle Komponente (B), das heißt also ein spezieller linearer hydroxyfunktioneller Polyester (B1), bevorzugt ein dihydroxyfunktioneller Polyester (B1), oder eine spezielle dihydroxyfunktionelle Komponente (B2), wird also unter Ausbildung einer Esterbindung mit einer entsprechenden carboxyfunktionellen endständigen Gruppe modifiziert, wodurch dann ein erfindungsgemäßes Poly- oder Diesterderivat entsteht.

Im Rahmen der vorliegenden Erfindung versteht sich also der Ausdruck "ringöffnende Umsetzung" so, dass außer genau dieser ringöffnenden Umsetzung keine weiteren Reaktionen auftreten, das heißt insbesondere keine weiteren Reaktionen der durch die ringöffnende Umsetzung entstehenden Carbonsäuregruppen mit weiteren, in der Reaktionsmischung noch vorhandenen Molekülen der hydroxyfunktionellen Komponente (B) auftreten. Wie der Fachmann weiß, wäre eine solche Kondensationsreaktion unter Ausbildung einer weiteren Esterbindung grundsätzlich möglich. Eine solche Weiterreaktion kann jedoch auf dem Fachmann ebenfalls bekannte Weise durch entsprechend angepasste Reaktionsführung problemlos unterbunden werden. Bekanntermaßen weist ein Anhydrid aufgrund der Ringspannung und der daraus folgenden höheren Energiefreisetzung bei der Reaktion mit einer Hydroxylgruppe eine höhere Reaktivität auf, als eine freie Carbonsäuregruppe. So ist es beispielsweise möglich, dass bei Reaktionstemperaturen gearbeitet wird, die die ringöffnende Umsetzung zulassen, jedoch keine weiteren Kondensationsreaktionen ermöglichen. Auch durch den gezielten Einsatz beziehungsweise das gezielte Weglassen, insbesondere das Weglassen, von Katalysatoren, beispielsweise typischen Säurekatalysatoren oder Dibutylzinnlaurat, kann die gewünschte Reaktionsführung gewährleistet werden. Auch der Zusatz oder das Weglassen von Wasser oder der Einsatz von beispielsweise Wasserabscheidern hat bekanntermaßen Einfluss auf die unterschiedliche Reaktivität, denn während bei der typischen Kondensationsreaktion zwischen Carbonsäure und Alkohol Wasser frei wird, ist dies bei der Reaktion von Anhydrid und Alkohol nicht der Fall. Der Fachmann weiß diese Bedingungen entsprechend anzupassen.

Durch die erfindungsgemäße ringöffnende Umsetzung entsteht demnach ein Poly- oder Diesterderivat, welches Carbonsäuregruppen beziehungsweise zusätzliche Carbonsäuregruppen enthält. Vorzugsweise weist das erfindungsgemäße Poly- oder Diesterderivat an beiden Enden entsprechende carboxyfunktionelle Gruppen auf.

Dies bedeutet also, dass bei der Umsetzung bevorzugt die Komponente (B) und/oder das molare Verhältnis des eingesetzten Anhydrids zu den Hydroxylgruppen der Komponente (B) so gewählt wird, dass das erfindungsgemäße Poly- oder Diesterderivat an beiden Enden entsprechende carboxyfunktionelle Gruppen trägt. Bevorzugt besitzt ein erfindungsgemäßes Poly- oder Diesterderivat genau zwei Carbonsäuregruppen pro Molekül.

Demnach ist bevorzugt, dass in den erfindungsgemäßen Poly- und Diesterderivaten die von der Komponente (B) stammende Moleküleinheit im Kern beziehungsweise im Zentrum des erfindungsgemäßen Poly- oder Diesterderivats liegt, während die aus dem Anhydrid stammende Moleküleinheiten als Seiten- beziehungsweise Endgruppen vorliegen. Besonders bevorzugt weisen die erfindungsgemäßen Poly- und Diesterderivate genau zwei Endgruppen auf, die aus dem Anhydrid hervorgehen. Dies bedeutet also insbesondere, dass die Komponente (B1) bevorzugt zwei endständige Hydroxylgruppen aufweist. Diese können dann beide mit dem Anhydrid (A) reagieren. Auch hinsichtlich der Komponente (B2) erfolgt damit bevorzugt eine komplette Umsetzung der beiden vorhandenen Hydroxylgruppen. Wie weiter oben beschrieben ist, enthält die Komponente (B) spezielle aliphatische Gruppen mit mindestens 12 Kohlenstoffatomen. Der daraus folgende hydrophobe Charakter der Komponente (B) trägt dann mit den an beiden Seiten endständig angebundenen Anhydridmolekülen beziehungsweise mit den beiden entsprechenden freien Carboxylgruppen, welche einen hydrophilen Charakter ausmachen, zu einem besonderen amphiphilen Charakter bei. Während die eher hydrophobe Einheit im Zentrum des Moleküls liegt, sind beide endständigen, sich gegenüber liegenden Seitengruppen hydrophil. Diese speziellen Poly- und Diesterderivate besitzen besonders vorteilhafte Eigenschaften hinsichtlich ihres flüssigkristallinen Verhaltens.

Bevorzugt werden bei der Umsetzung genau eine Art Anhydrid (A) und genau eine Art hydroxyfunktionelle Komponente (B) eingesetzt.

Wie der Fachmann weiß, entstehen bei den oben genannten Reaktionen und Reaktionsführungen aus rein statistischen Gründen auch Produkte, die nicht die beschriebene idealisierte Struktur aufweisen. Beispielsweise sind regelmäßig Nebenprodukte zu erwarten, die durch eine Weiterreaktion der entstandenen Poly- und Diesterderivate mit noch vorhandenen Molekülen der Komponente (B) entstehen oder Produkte, die trotz der bevorzugten beidseitigen Funktionalisierung nur einseitig funktionalisiert sind. Genauso möglich ist eine geringfügige Umsetzung des Anhydrids (A) mit Wasser beziehungsweise Luftfeuchte, wenn beispielsweise bei der Umsetzung nicht vollständig wasserfrei oder nicht unter Ausschluss von atmosphärischen Bedingungen (Luftfeuchte) gearbeitet wird. Auch nicht reagierte Ausgangskomponenten werden im Reaktionsgemisch vorhanden sein. Trotzdem können durch die beschriebene Reaktionsführung problemlos als Hauptprodukte die ebenfalls beschriebenen Poly- und Diesterderivate erhalten werden. Diese können auch ohne weitere Aufreinigung verwendet werden.

Die ringöffnende Umsetzung des Anhydrids (A) mit der Komponente (B) kann nach unterschiedlichen, dem Fachmann bekannten Methoden erfolgen, wobei die oben beschriebene Unterbindung der Weiterreaktion der gebildeten Poly- oder Diesterderivate mit Molekülen der hydroxyfunktionellen Komponente (B) zu beachten ist. Die Umsetzung kann beispielsweise in Masse oder in Lösung, bevorzugt in Lösung mit organischen Lösemitteln wie 4-Methyl-2-pentanon oder weiteren gängigen Lösemitteln, bei Temperaturen von beispielsweise 50°C bis 150°C, bevorzugt 60°C bis 125°C, insbesondere 65°C bis 100°C erfolgen. Insbesondere durch Reaktionstemperaturen von unter 100°C kann eine Weiterreaktion von Poly- oder Diesterderivaten mit Molekülen der hydroxyfunktionellen Komponente (B) effektiv unterbunden werden. Selbstverständlich können auch typische Katalysatoren wie Schwefelsäure oder Dibutylzinnlaurat eingesetzt werden, wobei allerdings das Weglassen solcher Katalysatoren von Vorteil ist, um die genannte Weiterreaktion zu unterbinden. Die Poly- und Diesterderivate können beispielsweise als Reaktionsgemisch mit organischen Lösemitteln oder nach Destillation von gegebenenfalls eingesetzten organischen Lösemitteln als feste Harze oder Öle erhalten werden.

Die Komponente (A) wird mit der Komponente (B) bei der ringöffnenden Umsetzung beziehungsweise der Herstellung der Poly- und Diesterderivate bevorzugt so umgesetzt, dass das molare Verhältnis der Anhydridgruppen der Komponente (A) zu den Hydroxylgruppen der Komponente (B) bei größer 0,6, besonders bevorzugt zwischen 0,7 und 2,0 liegt. In einer besonders vorteilhaften Ausführungsform der vorliegenden Erfindung wird so gearbeitet, dass die Anhydridgruppen der Komponente (A) im molaren Unterschuss zu den Hydroxylgruppen der Komponente (B) vorhanden sind. Darunter bevorzugt ist ein molares Verhältnis der Anhydridgruppen der Komponente (A) zu den Hydroxylgruppen der Komponente (B) von 0,7 bis kleiner 1,0, insbesondere 0,8 bis 0,95. Auf diese Weise gelingt ein vollständiger Umsatz des Anhydrids, sodass nach der Umsetzung praktisch keine Anhydridmoleküle in der Reaktionsmischung zurückbleiben. Die angegebenen molaren Verhältnisse verstehen sich als aus der jeweiligen Funktionalität der eingesetzten Ausgangsverbindungen errechnete Verhältnisse. Im Fall der Komponente (B1) wird also neben der eingesetzten Masse die gemessene OH-Zahl als Berechnungsgrundlage herangezogen. Bei den monomeren Ausgangsverbindungen (Komponente (B2), Anhydrid (A)) ergibt sich die molare Menge funktioneller Gruppen aus dem Molekulargewicht und der eingesetzten Masse.

Die Polyesterderivate weisen bevorzugt eine OH-Zahl von 0 bis 50, besonders bevorzugt von 2 bis 30, ganz besonders bevorzugt von 5 bis 25 mg KOH/g auf. Besonders bevorzugt weisen die Derivate also einen nur sehr geringen Anteil an Hydroxylgruppen auf, welcher beispielsweise auf einen rein synthesebedingten nicht quantitativen Umsatz zurückzuführen ist. Die Säurezahl der Polyesterderivate ist durch die aus dem Anhydrid resultierenden Carboxylgruppen bevorzugt höher und liegt besonders bevorzugt im Bereich von 50 bis 200 mg KOH/g, bevorzugt 70 bis 140 mg KOH/g.

Das zahlenmittlere Molekulargewicht der Polyesterderivate liegt bevorzugt im Bereich von 600 bis 3000 g/mol, insbesondere bevorzugt 900 bis 2000 g/mol, während das gewichtsmittlere Molekulargewicht bevorzugt im Bereich von 1600 bis 3600 g/mol, insbesondere bevorzugt 2000 bis 3000 g/mol liegt.

Aus dem oben Gesagten folgt, dass die Diesterderivate bevorzugt dicarboxyfunktionell sind, das heißt zwei Carboxylgruppen aufweisen. Demnach sind bevorzugt nur wenige Hydroxylgruppen in den Diesterderivaten vorhanden, die beispielsweise durch eine nicht völlig vollständige Umsetzung der Komponente (B2) zurückbleiben. Bevorzugt ist die OH-Zahl von 0 bis 50, besonders bevorzugt von 2 bis 30, ganz besonders bevorzugt von 5 bis 25 mg KOH/g. Dieser geringe Anteil an Hydroxylgruppen ist natürlich gleichbedeutend mit einer im Mittel etwas niedrigeren Anzahl von Carboxylgruppen pro Molekül, das heißt also insbesondere einer im Mittel geringfügig niedrigeren Anzahl als zwei Carboxylgruppen pro Molekül. Auch für die Diesterderivate lässt sich die Säurezahl wie oben beschrieben bestimmen und ist bevorzugt von 100 bis 250 mg KOH/g, besonders bevorzugt 125 bis 225 mg KOH/g, ganz besonders bevorzugt von 150 bis 200 mg KOH/g.

### Wässrige Zusammensetzung

Die erfindungsgemäße wässrige Zusammensetzung enthält mindestens ein erfindungsgemäßes Poly- und/oder Diesterderivat sowie Wasser. Bevorzugt bestehen die wässrigen Zusammensetzungen zu mindestens 80 Gew.-%, bevorzugt zu mindestens 90 Gew.-% aus mindestens einem erfindungsgemäßes Poly- und/oder Diesterderivat sowie Wasser. Enthalten sein können ferner beispielsweise Neutralisationsmittel, insbesondere die weiter unten beschriebenen Neutralisationsmittel.

Wässrig bedeutet im Rahmen der vorliegenden Erfindung, dass organische Lösemittel beispielsweise lediglich als Restanteile oder Verunreinigungen eingeschleppt werden, beispielsweise durch Restanteile organischer Lösemittel, die bei der Herstellung der Poly- und Diesterderivate eingesetzt wurden und sich gegebenenfalls nicht vollständig durch Destillation abtrennen ließen. Insbesondere ist mit wässrig gemeint, dass der Anteil organischer Lösemittel weniger als 5 Gew.-%, bevorzugt weniger als 2 Gew.-%, jeweils bezogen auf das Gesamtgewicht der wässrigen Zusammensetzung, beträgt. Ganz besonders bevorzugt ist die wässrige Zusammensetzung frei von organischen Lösemitteln. Insbesondere ist der Ausdruck "wässrig" bevorzugt so zu verstehen, dass die Zubereitung mindestens 30 Gew.-% Wasser, bevorzugt mindestens 35 Gew.-% Wasser, insbesondere 35 bis 80 Gew.-% Wasser und ganz besonders bevorzugt 45 bis 75 Gew.-% Wasser enthält.

Die Poly- und Diesterderivate lassen sich trotz der hydrophoben Gruppen, die aus der Komponente (B) stammen, aufgrund ihrer aus der Komponente (A) stammenden Carboxylgruppen und der daraus folgenden Eignung zur Anionenbildung in Wasser einbringen. Bekanntermaßen kann ein solcher Prozess durch gezielte Neutralisation von zur Anionbildung befähigten Gruppen, insbesondere Carboxylgruppen, effektiver gestaltet werden. Demnach werden die Carbonsäuregruppen der Poly- und Diesterderivate bevorzugt vor oder während der Zugabe von Wasser, bevorzugt vor der Zugabe von Wasser, mit entsprechenden Neutralisationsmitteln neutralisiert. Zur Neutralisation der Carboxylgruppen werden bevorzugt Ammoniak, Amine und/oder Aminoalkohole eingesetzt. Bevorzugt werden Di- und Triethylamin, Dimethylaminoethanol, Diisopropanolamin, Morpholine und/oder N-Alkylmorpholine eingesetzt.

Bevorzugt wird dabei das molare Verhältnis von aus dem Neutralisationsmittel zur Neutralisation befähigten Gruppen, insbesondere primären, sekundären und tertiären Aminogruppen, und Carbonsäuregruppen größer 0,7, bevorzugt zwischen 0,75 und 1,5, insbesondere zwischen 0,8 und 1,3 und ganz besonders bevorzugt zwischen 0,85 und 1,2 gewählt. Auf diese Weise kann ein signifikanter Anteil der Carbonsäuregruppen neutralisiert werden oder auch ein Neutralisationsgrad von annähernd 1 erreicht werden.

Der Anteil der Poly- und/oder Diesterderivate in den wässrigen Zusammensetzungen, bezogen auf die Gesamtmenge der Zusammensetzung liegt beispielsweise im Bereich von 20 bis 65 Gew.-%, bevorzugt 25 bis 55 Gew.-%. Der Anteil der Poly- oder Diesterderivate wird über den Festkörper bestimmt (Bestimmungsmethode siehe oben).

Die erfindungsgemäßen Poly- und Diesterderivate bilden mit Wasser flüssigkristalline Phasen.

Die flüssigkristallinen Phasen können sich in den wässrigen Zusammensetzungen spontan ausbilden, das heißt durch Zugabe des Poly- und/oder Diesterderivats zu Wasser werden flüssigkristalline Phasen ausgebildet, sodass wässrige Zusammensetzungen enthaltend flüssigkristalline Phasen entstehen. Möglich ist auch, dass die flüssigkristallinen Phasen durch gezielte Verdünnung der Poly- und Diesterderivate in Wasser ausgebildet werden, das heißt also durch spezifische Einstellung eines bestimmten Anteils des Poly- oder Diesterderivats in Wasser, insbesondere in den oben angegebenen Anteilsbereichen

Ein bevorzugtes Verfahren zur Herstellung von flüssigkristallinen Phasen wird in der Folge beschrieben.

Die Poly- oder Diesterderivate, welche nach ihrer Herstellung beispielsweise als festes Harz oder aber in Mischung mit organischen Lösemitteln vorliegen, werden mit Neutralisationsmitteln versetzt. Daran anschließend wird das organische Lösemittel, sofern vorhanden, abdestilliert und Wasser hinzugegeben. Das zugegebene Wasser kann dabei Raumtemperatur haben, das heißt also eine Temperatur zwischen 18 und 25°C aufweisen, oder aber auch leicht temperiert sein, beispielsweise auf 50 bis 70°C. Bevorzugt ist die Temperatur des Wassers auf 18 bis 70°C eingestellt. Das Wasser wird dabei bevorzugt tropfenweise zugegeben, sodass die Poly- oder Diesterderivate sukzessive mit Wasser verdünnt werden. Dabei wird dann auf den gewünschten Gehalt an Poly- oder Diesterderivat eingestellt, insbesondere werden die oben genannten bevorzugten Gehalte beziehungsweise Anteile eingestellt.

Alternativ kann nach der Zugabe des Neutralisationsmittels auch erst eine initiale Menge Wasser hinzugegeben werden und dann das gegebenenfalls vorhandene organische Lösemittel abdestilliert werden. Die initiale Menge des Wassers wird bevorzugt so gewählt, dass nach der Zugabe des Wassers und dem Abdestillieren der gegebenenfalls vorhandenen organischen Lösemittel ein Anteil des Poly- oder Diesterderivats von 70 bis 85 Gew.-% resultiert. Das Wasser weist dabei bevorzugt ebenfalls eine Temperatur von 18 bis 70°C auf. Danach erfolgt dann wieder die sukzessive Verdünnung auf den gewünschten Anteil (Festkörper beziehungsweise nichtflüchtigen Anteil) des Poly- oder Diesterderivats.

Insbesondere bei einem Gehalt des Poly- oder Diesterderivats von 20 bis 65 Gew.-%, bevorzugt 25 bis 55 Gew.-%, weisen die wässrigen Zusammensetzungen besonders ausgeprägte flüssigkristalline Eigenschaften auf, das heißt also die wässrigen Zusammensetzungen enthalten dann ausgeprägte flüssigkristalline Phasen.

In einer weiteren besonderen Ausführungsform der vorliegenden Erfindung enthalten die wässrigen Zusammensetzungen noch schichtförmige Hydroxide, insbesondere schichtförmige Doppelhydroxide. Der Gehalt dieser schichtförmigen Hydroxide liegt beispielsweise von 2 bis 20 Gew.-%, ganz besonders bevorzugt 3 bis 10 Gew.-%.

Schichtförmige Hydroxide, insbesondere Doppelhydroxide (layered double hydroxides, LDH) und deren Herstellung sind dem Fachmann bekannt und werden beispielsweise in WO 2013056846 A1 beschrieben.

Die im Rahmen der vorliegenden Erfindung vorteilhaft einzusetzenden LDH werden beschrieben durch die Formel (II):

[M²⁺₍₁₋ₓ₎M³⁺ₓ(OH)₂][A^{y-}_{(x/y)}]·*n*H₂O (II)

wobei
M²⁺ für zweiwertige metallische Kationen steht und die M²⁺ bevorzugt ausgewählt werden aus der Gruppe bestehend aus Zn²⁺, Mg²⁺, Ca²⁺, Cu²⁺, Ni²⁺, Co²⁺, Fe²⁺, Mn²⁺, Cd²⁺, Pb²⁺, Sr²⁺ und Mischungen davon, bevorzugt Zn²⁺, Mg²⁺, Ca²⁺ und Mischungen davon, ganz besonders bevorzugt Zn²⁺ und/oder Mg²⁺, insbesondere Zn²⁺,
M³⁺ für dreiwertige metallische Kationen steht und die M³⁺ bevorzugt ausgewählt werden aus der Gruppe bestehend aus Al³⁺, Bi³⁺, Fe³⁺, Cr³⁺, Ga³⁺, Ni³⁺, Co³⁺, Mn³⁺, V³⁺, Ce³⁺, La³⁺ und Mischungen davon, bevorzugt Al³⁺, Bi³⁺ und/oder Fe³⁺, insbesondere Al³⁺,
A^{y-} für organische und anorganische Anionen steht und die A^{y-} bevorzugt ausgewählt werden aus der Gruppe bestehend aus Karbonat, Chlorid, Nitrat, Hydroxid, Bromid, Molybdat, Chromat, Salicylat, Oxalat, 2,4-Dimercapto-1,3,4-thiadiazol und dessen Derivate, EDTA, Benzotriazolat, organische Anionen von Aminosäuren, insbesondere alpha-Aminosäuren, bevorzugt Alanin, Arginin, Asparagin, Asparaginsäure, Cystein, Cystin Glutamin, Glutaminsäure, Histidin, Isoleucin Leucin, Lysin, Methionin, Phenylalanin, Prolin, Serin, Threonin, Tryptophan, Tyrosin, Valin, Selenocystein, Pyrrolysin und Selenomethionin sowie organische Anionen der folgenden Formel (III): 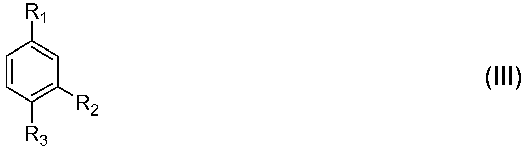 mit
   R₁ = COO⁻, SO₃⁻,
   R₂/ R₃ = NH₂, OH, H, wobei bevorzugte R₂ = R₃ = H ausgeschlossen ist,
   x einen Wert von 0,05 bis 0,5, insbesondere 0,15 bis 0,4, ganz besonders bevorzugt von 0,25 bis 0,35 und
   n einen Wert von 0 bis 10 einnimmt.

Besonders bevorzugt Anionen sind organische Anionen, insbesondere organische Anionen von alpha-Aminosäuren und solche der Formel (III).

Die flüssigkristallinen Phasen bilden aufgrund ihrer Flüssigkristallinität und der damit vorhandenen Balance zwischen Anisotropie und Fließfähigkeit sowie den damit zu korrelierenden weiteren anwendungstechnischen Eigenschaften, beispielsweise Einflüssen auf Pigmentorientierung und Rheologie von wässrigen Systemen bei gleichzeitig guter Formulierbarkeit, eine vielversprechende Grundlage für unterschiedliche Anwendungen.

Als potentielle Verwendungen zu nennen sind beispielsweise wässrige Automobil- und Industriebeschichtungen, die oft einen in hohem Maße vom Betrachtungswinkel abhängigen Helligkeits- und/oder Farbeffekt aufweisen sollen und deren flüssige Lackmaterialen einen hohen nichtflüchtigen Anteil aufweisen sollen, zugleich aber mit hohen Ausflussraten versprüht werden sollen. Gerade in diesem Zusammenhang ist der Einfluss auf Pigmentorientierung und gleichzeitig guter Formulierbarkeit durch die Flüssigkristallinität besonders nutzbringend. Andere Anwendungen stellen organische, korrosionsschützende Beschichtungen für metallische Substrate dar, die eine hohe Diffusionsbarriere gegenüber Sauerstoff und Feuchtigkeit (Barriere) mit einer effektiven Energiedissipation im Falle eindringender Projektile (beispielsweise Steinschlag bei Fahrzeugen) kombinieren sollen. Da einige der anisotropen Flüssigkeiten aus ausgedehnten, porösen Lamellen bestehen können, deren Porengröße definiert im Bereich von wenigen Nanometern liegen, sind diese auch als einfach zugängliche Ausgangsformulierungen für Trennmembranen denkbar.

Im Folgenden wird die Erfindung anhand von Beispielen weiter erläutert.

### Beispiele

Alle im Rahmen der Beispiele angegebenen Messdaten wurden, sofern nicht anders angegeben, nach den in der allgemeinen Beschreibung genannten Bestimmungsmethoden erhalten.

### 1. Herstellung von erfindungsgemäßen Polyester- und Diesterderivaten

### 1.1 Herstellung eines Polyesters (B1)

Ein Polyester (B1) wird wie folgt hergestellt. In einen Reaktor mit Ankerrührer, Stickstoffeinlass und Wasserabscheider mit Kopftemperaturmesseinrichtung und aufgesetzter Füllkörperkolonne mit Rückflusskühler werden 44,40 g vollständig hydriertes Bisphenol-A, 7,07 g Cyclohexan-1,2-dicarbonsäureanhydrid, 23,41 g dimere Fettsäure (Pripol®1012, Firma Unichema, basierend auf ungesättigten C-18-Fettsäurederivaten mit einem Dimerengehalt von mindestens 97 Gew.-%, Trimerengehalt höchstens 1 Gew.-%, Monomerengehalt höchstens Spuren) eingebracht. Der Reaktorinhalt wird in einer Stickstoffatmosphäre unter Rühren solange auf 190 Grad C erhitzt bis die Reaktionsmischung eine Säurezahl von 8-12 mg KOH/g erreicht. Die Kopftemperatur sollte 100 °C nicht überschreiten. Nach 9 Tagen ist eine Säurezahl von 11,4 mg KOH/ g erreicht und die Reaktionsmischung wird abgekühlt. Es werden 1,7 ml Wasser aus der Kondensationsreaktion aufgefangen. Die Ausbeute beträgt 72,7 g festes Harz. Das Harz besitzt eine OH-Zahl von 143 mg KOH/g, ein massenmittleres Molekulargewicht von 1884 g/mol und ein zahlenmittleres Molekulargewicht von 753 g/mol.

### 1.2 Herstellung eines erfindungsgemäßen Polyesterderivats EP1

Ein erfindungsgemäßes Polyesterderivat EP1 wird wie folgt hergestellt. Zur Harzschmelze des in Beispiel 1.1 erhaltenen Polyesters (B1) werden 27 g 2-Butanon zugegeben und in einem Reaktor mit Ankerrührer, Stickstoffeinlass und Rückflusskühler unter Rühren gelöst und 35,7 g 2-Octenylbernsteinsäureanhydrid (0,9 Äquivalente in Bezug auf die molare Menge an Hydroxylgruppen im eingesetzten Polyester (B1)) zugegeben. Die Mischung wird in einer Stickstoffatmosphäre auf 95 Grad C erhitzt und unter Rückfluss bei dieser Temperatur 18 Stunden gerührt. Die so erhaltene klare farblose Lösung besitzt einen nichtflüchtigen Anteil von 68,4 Gew.-% (Anteil des Polyesterderivats EP1). Die Säurezahl beträgt 97 mg KOH/g. Das Harz besitzt eine OH-Zahl von 18 mg KOH/g, ein gewichtsmittleres Molekulargewicht von 2218 g/mol und ein zahlenmittleres Molekulargewicht von 1181 g/mol.

### 1.3 Herstellung eines erfindungsgemäßen Diesterderivats ED1

Ein erfindungsgemäßes Diesterderivat ED1 wird wie folgt hergestellt. 12,6 g vollständig hydriertes Bisphenol-A (Komponente (B2)) werden in einem Reaktor mit Ankerrührer, Stickstoffeinlass und Rückflusskühler in 23 g 2-Butanon gelöst und 20,4 g n-Octenylbernsteinsäureanhydrid (0,9 Äquivalente in Bezug auf die molare Menge an Hydroxylgruppen im hydrierten Bisphenol-A) zugegeben. Die Mischung wird in einer Stickstoffatmosphäre auf 95 Grad C erhitzt und unter Rückfluss bei dieser Temperatur 22 Stunden gerührt. Die so erhaltene klare farblose Lösung besitzt einen nichtflüchtigen Anteil von 70,1 Gew.-% (Anteil des Diesterderivats ED1). Die Säurezahl beträgt 175 mg KOH/ g. Das Harz besitzt eine OH-Zahl von 27 mg KOH/g, ein gewichtsmittleres Molekulargewicht von 841 g/mol und ein zahlenmittleres Molekulargewicht von 701 g/mol.

### 2. Herstellung eines nicht erfindungsgemäßen Polyesterderivats VP1

Das Polyesterderivat VP1 wird wie folgt hergestellt. Zur Harzschmelze des in Beispiel 1.1 erhaltenen Polyesters (B1) werden 8,35 g 1,2,4-Benzoltricarbonsäureanhydrid hinzugefügt. Der Reaktor wird mit Wasserabscheider und Rückflusskühler, ohne Füllkörperkolonne, betrieben. Das Gemisch wird auf 160 Grad C aufgeheizt und bei dieser Temperatur 25 Stunden gerührt. Die Ausbeute beträgt 79,02 g festes Harz. Die Säurezahl beträgt 32,5 mg KOH/g. Es können 0,6 ml Wasser durch die Kondensationsreaktion aufgefangen werden. Das Harz besitzt eine OH-Zahl von 68 mg KOH/g, ein gewichtsmittleres Molekulargewicht von 7188 g/mol und ein zahlenmittleres Molekulargewicht von 2171 g/mol.

### 3. Herstellung von wässrigen Zusammensetzungen sowie flüssigkristallinen Phasen

### 3.1 Herstellung von erfindungsgemäßen wässrigen Zusammensetzungen enthaltend EP1

Die in Beispiel 1.2 erhaltene Lösung (enthaltend EP1) wird in einem Reaktor mit Ankerrührer, Stickstoffeinlass, Rückflusskühler und Destillationsbrücke unter Stickstoff und Rühren auf 80 Grad C erwärmt und tropfenweise mit 10,19 g N,N-Dimethylamino-2-ethanol versetzt, entsprechend einem Neutralisationsgrad von 0,95 der über die Säurezahl ermittelten Carboxylgruppen des Polyesterderivats. Nach weiterem zehnminütigem Rühren wird das 2-Butanon bei 80 Grad C unter Vakuum abdestilliert bis ein Gehalt von 2-Butanon in dem verbleibenden Polyesterderivat von < 0,5 Gew.-% (bestimmt über gaschromatographische Analyse) erreicht ist.

Zu dieser Harzschmelze wird tropfenweise deionisiertes, auf Raumtemperatur temperiertes, Wasser zugegeben. Das Polyesterderivat EP1 wird von den (annähernd) 100 Gew.-% zunächst mit einem Massenstrom von 2 g pro Minute auf einen nichtflüchtigen Anteil von 42 Gew-% herunter verdünnt. Danach wird 20 Minuten nachgerührt und eine Probe der entsprechenden wässrigen Zusammensetzung für die unter Punkt 4 beschriebene Untersuchung abgetrennt (wässrige Zusammensetzung E1). Anschließend wird die verbleibende Dispersion wiederum durch tropfenweise Zugabe von deionisiertem, auf Raumtemperatur temperiertem Wasser mit einem Massenstrom von 2 g pro Minute auf einen nichtflüchtigen Anteil von 33 Gew-% herunterverdünnt (wässrige Zusammensetzung E2). Die wässrigen Zusammensetzungen werden auf Raumtemperatur abgekühlt und für die weitere Charakterisierung (Punkt 4) für 3 Tage zur Seite gestellt.

### 3.2 Herstellung von erfindungsgemäßen wässrigen Zusammensetzungen enthaltend ED1

Die in Beispiel 1.3 erhaltene Lösung (enthaltend ED1) wird in einem Reaktor mit Ankerrührer, Stickstoffeinlass, Rückflusskühler und Destillationsbrücke unter Stickstoff und Rühren auf 80 Grad C erwärmt und tropfenweise mit 12,4 g N,N-Dimethylamino-2-ethanol versetzt, entsprechend einem Neutralisationsgrad von 0,95 der über die Säurezahl ermittelten Carboxylgruppen des Diesterderivats. Anschließend werden 24,3 g Wasser unter Rühren zugegeben. Das 2-Butanon wird bei 80 Grad C unter Vakuum abdestilliert bis ein Gehalt von 2-Butanon in dem verbleibenden Diesterderivat von < 0,5 Gew.-% (bestimmt über gaschromatographische Analyse) erreicht ist.

Diese Dispersion besitzt einen nichtflüchtigen Anteil von 76 Gew.-% und wird mit Wasser durch tropfenweise Zugabe mit einem Massenstrom von 2 g pro Minute bei 60 Grad C auf einen nicht flüchtigen Anteil von 40 Gew.-% herunterverdünnt (wässrige Zusammensetzung E3). Die wässrigen Zusammensetzungen werden auf Raumtemperatur abgekühlt und für die weitere Charakterisierung (Punkt 4) für 3 Tage zur Seite gestellt.

### 3.3 Herstellung von nicht erfindungsgemäßen wässrigen Zusammensetzungen enthaltend VP1

Das in Beispiel 2 erhaltene Harz wird in einem Reaktor mit Ankerrührer, Stickstoffeinlass, Rückflusskühler und Destillationsbrücke mit 33,5 g 4-Methylpentan-2-on versetzt und über einen Zeitraum von 5 Tagen zum vollständigen Lösen stehen gelassen. Das so erhaltene Gemisch wird unter Stickstoffatmosphäre und Rühren auf 90 Grad C erwärmt und innerhalb von 10 Minuten mit 3,9 g N,N-Dimethylamino-2-ethanol versetzt, entsprechend einem Neutralisationsgrad von 0,95 der über die Säurezahl ermittelten Carboxylgruppen des Polyesterderivats. Nach Abkühlen auf 80 Grad C werden innerhalb von 120 Minuten 47,8 g Wasser unter Rühren zugegeben. Das 4-Methylpentan-2-on wird bei 73 Grad C unter Vakuum abdestilliert bis ein Gehalt von 4-Methylpentan-2-on in dem verbleibenden Polyesterderivat von < 0,5 Gew.-% (bestimmt über gaschromatographische Analyse) erreicht ist.

Diese Dispersion besitzt einen nichtflüchtigen Anteil von 61 Gew.-% und wird mit Wasser durch tropfenweise Zugabe mit einem Massenstrom von 2 g pro Minute bei 73 Grad C auf einen nichtflüchtigen Anteil von 36 Gew.-% herunterverdünnt. Die Temperatur von 73 Grad C wird 4 Stunden gehalten und die Dispersion unter ständigem Rühren homogenisiert (wässrige Zusammensetzung V1). Die wässrigen Zusammensetzungen werden auf Raumtemperatur abgekühlt und für die weitere Charakterisierung (Punkt 4) 3 Tage zur Seite gestellt.

Tabelle 1 gibt nochmals einen Überblick über die hergestellten wässrigen Zusammensetzungen

**Tabelle 1:**

| **Wässrige Zusammensetzung** | **Enthaltenes Derivat** | **Anteil Derivat (nichtflüchtiger Anteil) in Gew.-%** |
|---|---|---|
| E1 | EP1 | 42 |
| E2 | EP1 | 33 |
| E3 | ED1 | 40 |
| V1 | VP1 | 36 |

### 4. Anwendungstechnische Untersuchung der wässrigen Zusammensetzungen

Die unter Punkt 3 erhaltenen wässrigen Zusammensetzungen wurden wie folgt untersucht.

### 4.1 Optische Mikroskopie unter gekreuzt polarisiertem Licht (OM)

Mit der OM kann das an sich bekannte Phänomen der Doppelbrechung detektiert werden. Während bei doppelbrechenden Phasen, beispielsweise kristallinen Phasen, bei der gekreuzten Polarisatoranordnung (Primärfilter beziehungsweise Linearpolarisator des Lichtes der Lichtquelle sowie hierzu um 90° gedrehter Sekundärfilter beziehungsweise Analysator) Licht in den Analysator dringt und somit entsprechend helle Bereiche auf den erhaltenen Mikroskopiebildern zu sehen sind, ist dies bei rein isotropen Phasen nicht der Fall. Bei letzteren findet also eine vollständige Auslöschung statt, die Bilder sind dunkel beziehungsweise komplett schwarz. Sofern also helle Bereiche zu sehen sind, kann auf einen kristallinen (anisotropen, geordneten) Charakter geschlossen werden.

Die OM wurde an einem Olympus BX 51 Mikroskop mit XC 10 Digitalkamera und UM Plan FI-Objektiven aufgenommen. Dabei wurde eine Probe der jeweiligen wässrigen Zusammensetzung (E1 bis E3, V1) zwischen einem Objektträger und einem Deckglas präpariert. Die Probe wird durch aufeinanderdrücken von Objektträger und Deckglas auf ein Volumen von etwa 100 µm gebracht und mindestens eine Minute ruhen gelassen, um auf diese Weise die geringfügig gescherte Probe relaxieren zu lassen. Die Messung erfolgt bei Raumtemperatur mit 90 Grad gekreuzten Polarisationsfiltern im Durchlicht.

Die Proben E1 bis E3 (erfindungsgemäße wässrige Zusammensetzungen) zeigen unter dem Mikroskop deutlich ausgeprägte Bereiche, in denen Licht durch den Analysator dringt. Die Doppelbrechung der erfindungsgemäßen Phasen zeigt, dass anisotrope geordnete Strukturen vorliegen, deren "oily streak" Texturen zu lamellaren flüssigkristallinen Phasen passen. Die Vergleichsprobe V1 zeigt ebenfalls eine Doppelbrechung.

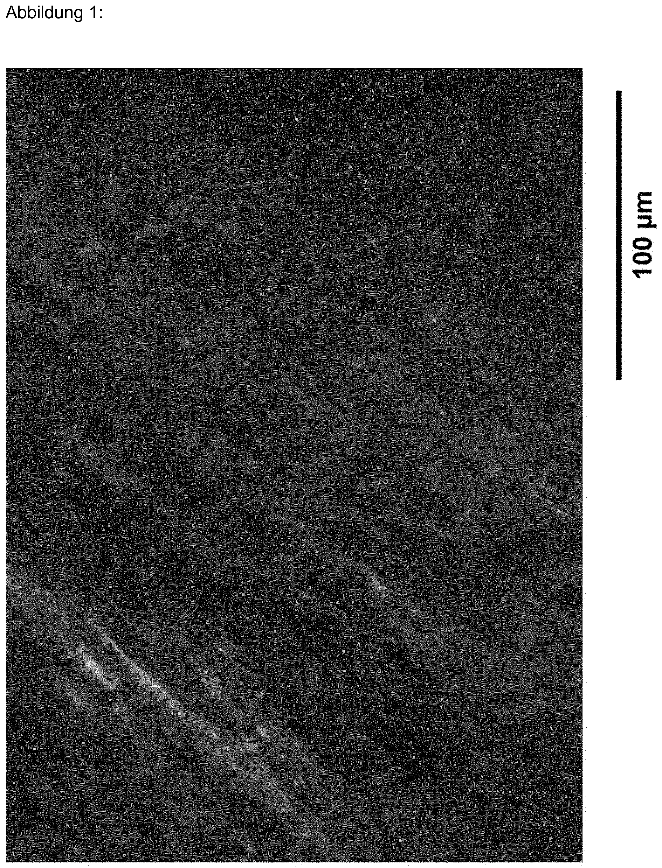

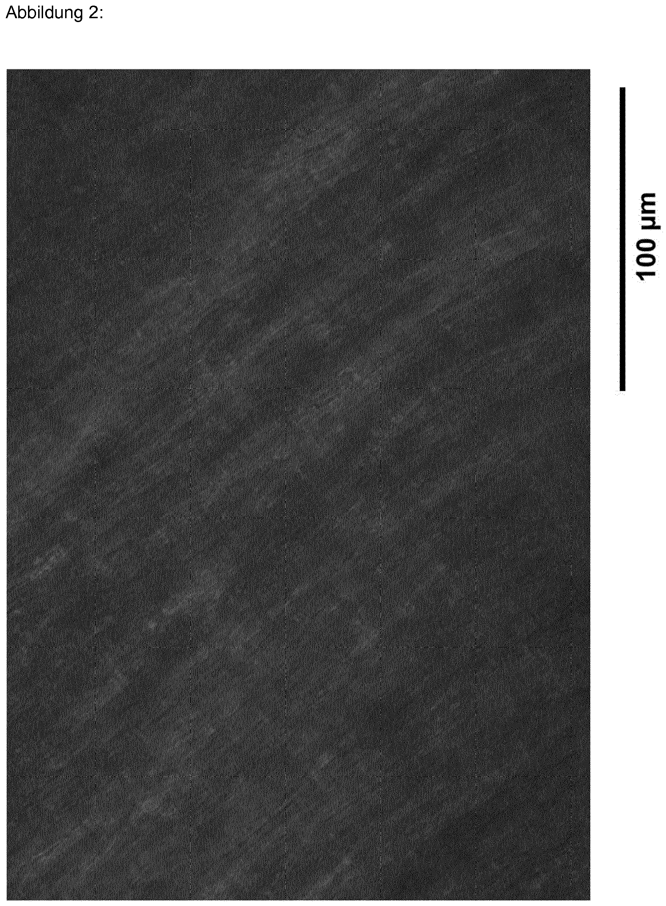

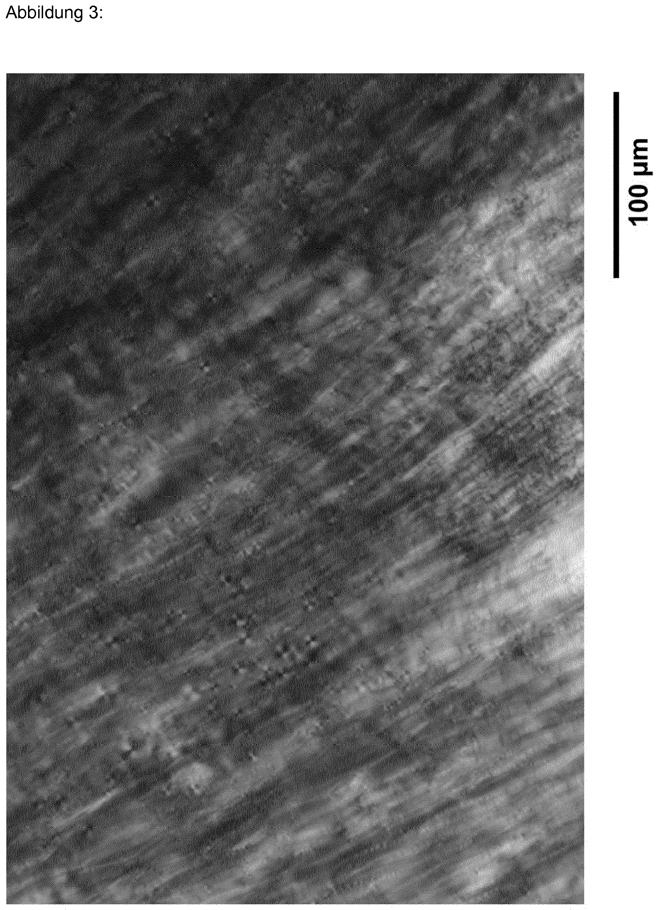

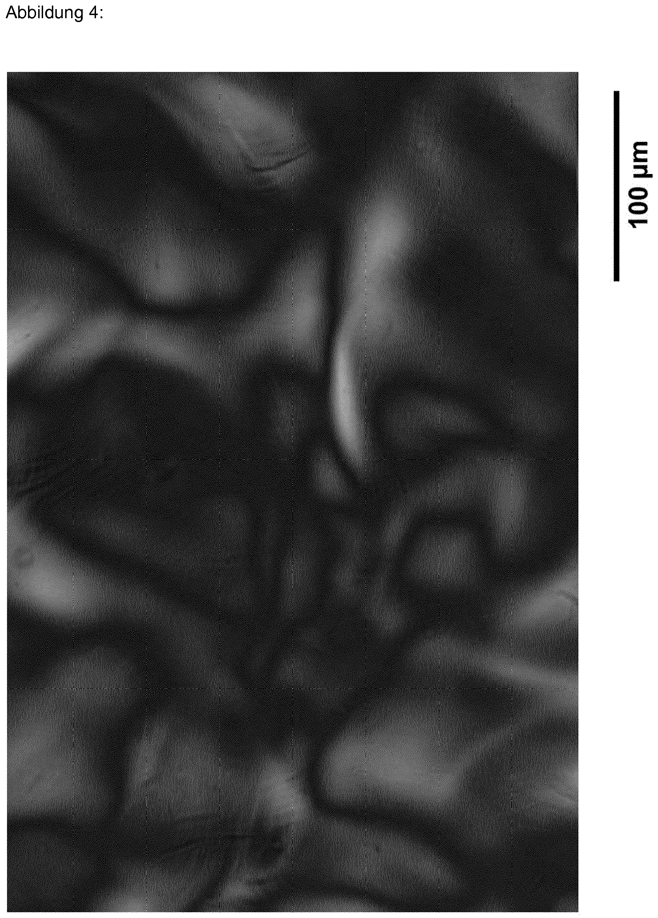

Die genannten Eigenschaften sind nochmals den Abbildungen 1 bis 4 zu entnehmen.

Alle vier wässrigen Zusammensetzungen zeigen also geordnete, anisotrope Phasen.

### 4.2 Rheologische Charakterisierung der wässrigen Zusammensetzungen

Anhand der wässrigen Zusammensetzungen E1 bis E3 und V1 wurden so genannte Amplitudensweeps bei 23°C an einem Anton Paar MCR501 mit einer CP50/1 Messgeometrie über einem Schubspannungsbereich von 0,2 Pa (beziehungsweise 0,5/2 Pa) bis 100 Pa durchgeführt und dabei das Speichermodul G' und das Verlustmodul G" der jeweiligen wässrigen Zusammensetzung gemessen.

Während das Speichermodul G' Ausdruck der Elastizität einer Probe ist (das heißt also die elastischen, beispielsweise gelartigen Eigenschaften eine Probe und damit die nach Energieeinbringung erfolgende elastische Verformung widerspiegelt), spiegelt das Verlustmodul G" den dissipativ freigesetzten beziehungsweise verlorengegangenen viskosen Anteil der in die Probe eingebrachten Energie wider und ist somit grundsätzlich Ausdruck eines fluiden Charakters.

Systeme, bei denen das Speichermodul G' deutlich größer ist als das Verlustmodul G", weisen praktisch keinerlei fluiden Charakter auf, sind also insbesondere nicht fließfähig. Im Gegenteil zu einem fluiden und fließfähigen Charakter haben solche Systeme in der Regel gelartigen Charakter, der insbesondere zu einer deutlich erschwerten Formulierbarkeit und Einsatzfähigkeit führt. So können solche gelartigen Systeme beispielsweise nur schwerlich homogene Mischungen mit anderen Komponenten bilden.

Systeme, bei denen das Verlustmodul G" größer als das Speichermodul G' ist, sind in der Regel fließfähige und damit gut zu formulierende Systeme. Dasselbe gilt für Systeme, bei denen das Verlustmodul G" und das Speichermodul G' in einer ähnlichen Größenordnung liegen. Solche, als viskoelastische Flüssigkeiten bekannte Systeme weisen ebenfalls den für eine gute Formulierbarkeit notwendigen fluiden Charakter auf.

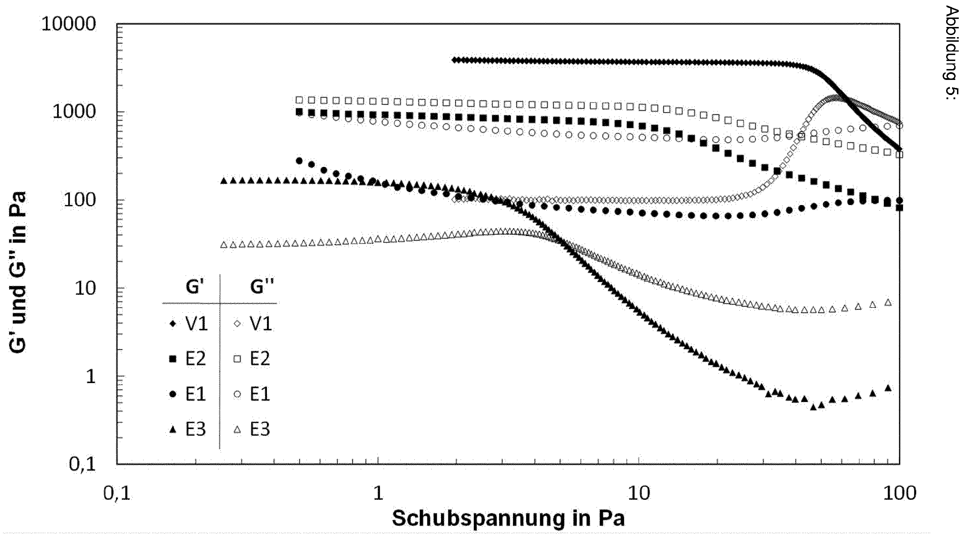

Die erfindungsgemäßen Zusammensetzungen weisen über einen breiten Schubspannungsbereich (0,2 bis 100 Pa) Verlustmodule G" und Speichermodule G' in etwa derselben Größenordnung auf. So unterscheiden sich beispielsweise die Beträge von Verlustmodul G" und Speichermodul G' bei einer Schubspannung von 10 Pa um nur etwa 442 Pa (Zusammensetzung E1), 434 Pa (Zusammensetzung E2) und 34 Pa (Zusammensetzung E3), wobei jeweils das Verlustmodul G" größer als das Speichermodul G' ist. Ähnliche Verhältnisse ergeben sich auch bei höheren oder niedrigeren Schubspannungen. Die Module liegen jeweils in ähnlicher Größenordnung, wobei für die Zusammensetzungen E1 und E2 das Verlustmodul G" über den gesamten Bereich größer als das Speichermodul G' ist und für die Zusammensetzung E3 bei Schubspannungen von unter 5 Pa das Speichermodul nur geringfügig größer ist als das Verlustmodul (Abbildung 5 zeigt das Verlustmodul und das Speichermodul der gemessenen Proben über den gesamten gemessenen Bereich der Schubspannung).

Diese Daten passen optimal zu den rein optisch erfassbaren makroskopischen Zuständen der Zusammensetzungen. Alle drei Proben sind fließfähig und damit gut formulierbar. Sie vereinen also einen kristallinen, anisotropen und geordneten Zustand mit einer ausgeprägten Fließfähigkeit und einer daraus folgenden guten Formulierbarkeit. Sie sind also flüssigkristallin.

Im Gegenteil dazu ist bei der nicht erfindungsgemäßen Probe V1 über einen weiten Schubspannungsbereich ein deutlich größeres Speichermodul G' als Verlustmodul G" zu beobachten. Bei einer Schubspannung von 10 Pa ist das Speichermodul um etwa 3700 Pa größer als das Verlustmodul. Die Differenz ist hier also signifikant größer als bei den erfindungsgemäßen Systemen.

Wiederum passen die Daten optimal zum rein optisch erfassbaren makroskopischen Zustand der Zusammensetzung. Die Zusammensetzung V1 ist ein nicht fließfähiges Gel, welches sich nicht mit üblichen Laborrühren oder auch größeren Rührgeräten wie Dissolvern verarbeiten lässt und entsprechend eine sehr schlechte Formulierbarkeit aufweist.

Es zeigt sich also, dass die erfindungsgemäßen Zusammensetzungen über einen weiten Bereich von mechanischem Stress (Schubspannung) ihren flüssigkristallinen Charakter bewahren, während die nicht erfindungsgemäße Zusammensetzung einen gelartigen Charakter hat.

### Kurzbeschreibung der Abbildungen

### Abbildung 1:

Mikroskopiebild (optische Mikroskopie unter gekreuzt polarisiertem Licht) der wässrigen Zubereitung E1.

### Abbildung 2:

Mikroskopiebild (optische Mikroskopie unter gekreuzt polarisiertem Licht) der wässrigen Zubereitung E2.

### Abbildung 3:

Mikroskopiebild (optische Mikroskopie unter gekreuzt polarisiertem Licht) der wässrigen Zubereitung E3.

### Abbildung 4:

Mikroskopiebild (optische Mikroskopie unter gekreuzt polarisiertem Licht) der wässrigen Zubereitung V1.

### Abbildung 5:

Speichermodul (G') und Verlustmodul (G") von wässrigen Zusammensetzungen E1, E2, E3 und V1, aufgetragen gegen die Schubspannung.

## Patentansprüche

1. Carboxyfunktionelle Poly- und Diesterderivate herstellbar durch ringöffnende Umsetzung von
(A) mindestens einem Anhydrid der Formel (I) 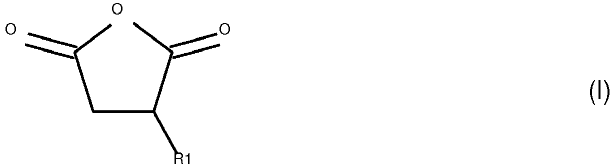 wobei R₁ = H, C₁- bis C₄₈-Alkyl, C₂- bis C₄₈-Alkenyl
mit
(B) mindestens einer hydroxyfunktionellen Komponente, wobei
(B1) zur Herstellung der Polyesterderivate mindestens ein linearer Polyester der zwei endständige Hydroxylgruppen aufweist, bei dessen Herstellung 7 bis 95 mol-%, bezogen auf die Gesamtmenge der bei der Herstellung des Polyesters (B1) eingesetzten Monomere, mindestens ein difunktionelles Monomer (b1) mit aliphatischen Gruppen mit 12 bis 70 Kohlenstoffatomen zwischen den funktionellen Gruppen eingesetzt wird,
und
(B2) zur Herstellung der Diesterderivate mindestens eine dihydroxyfunktionelle Komponente, welche eine aliphatische Gruppe mit 12 bis 70 Kohlenstoffatomen zwischen den Hydroxylgruppen aufweist,
eingesetzt wird.

2. Carboxyfunktionelle Poly- und Diesterderivate nach Anspruch 1, **dadurch gekennzeichnet, dass** für die Formel (I) R₁ = H und/oder C₆- bis C₄₈-Alkenyl gilt.

3. Carboxyfunktionelle Poly- und Diesterderivate nach Anspruch 1 oder 2, **dadurch gekennzeichnet, dass** die aliphatischen Gruppen der Monomere (b1) und der Komponente (B2) 13 bis 50 Kohlenstoffatome aufweisen.

4. Carboxyfunktionelle Poly- und Diesterderivate nach einem oder mehreren der Ansprüche 1 bis 3, **dadurch gekennzeichnet, dass** die Monomere (b1) ausgewählt werden aus der Gruppe bestehend aus vollständig hydrierten Bisphenolen, dimeren aliphatischen Fettalkoholen und dimeren aliphatische Fettsäuren.

5. Carboxyfunktionelle Poly- und Diesterderivate nach einem oder mehreren der Ansprüche 1 bis 4, **dadurch gekennzeichnet, dass** der Polyester (B1) eine Hydroxylzahl von 80 bis 200 mg KOH/g aufweist.

6. Carboxyfunktionelle Poly- und Diesterderivate nach einem oder mehreren der Ansprüche 1 bis 5, **dadurch gekennzeichnet, dass** die Komponente (B2) ausgewählt wird aus der Gruppe bestehend aus vollständig hydrierten Bisphenolen und/oder dimeren aliphatischen Fettalkoholen.

7. Carboxyfunktionelle Poly- und Diesterderivate nach einem oder mehreren der Ansprüche 1 bis 6, **dadurch gekennzeichnet, dass** das molare Verhältnis der Anhydridgruppen der Komponente (A) zu den Hydroxylgruppen der Komponente (B) bei der ringöffnenden Umsetzung von 0,7 bis kleiner 1,0 liegt.

8. Carboxyfunktionelle Poly- und Diesterderivate nach einem oder mehreren der Ansprüche 1 bis 7, **dadurch gekennzeichnet, dass** sie eine Säurezahl zwischen 50 und 250 mg KOH/g aufweisen.

9. Carboxyfunktionelle Poly- und Diesterderivate nach einem oder mehreren der Ansprüche 1 bis 8, **dadurch gekennzeichnet, dass** sie eine OH-Zahl von 2 bis 30 mg KOH/g aufweisen.

10. Carboxyfunktionelle Poly- und Diesterderivate nach einem oder mehreren der Ansprüche 1 bis 9, **dadurch gekennzeichnet, dass** bei der Herstellung der Polyester (B1) 60 bis 90 mol-%, bezogen auf die Gesamtmenge der bei der Herstellung des Polyesters (B1) eingesetzten Monomere, mindestens ein Monomer (b1) eingesetzt wird.

11. Verfahren zur Herstellung von Poly- und Diesterderivaten gemäß einem oder mehreren der Ansprüche 1 bis 10, bei dem
(A) mindestens ein Anhydrid der Formel (I) 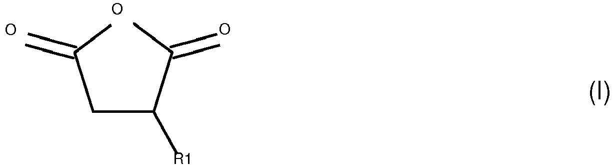 wobei R₁ = H, C₁- bis C₄₈-Alkyl, C₂- bis C₄₈-Alkenyl
mit
(B) mindestens einer hydroxyfunktionellen Komponente unter Öffnung des Anhydridrings umgesetzt wird, wobei
(B1) zur Herstellung der Polyesterderivate mindestens ein linearer Polyester der zwei endständige Hydroxylgruppen aufweist, bei dessen Herstellung 7 bis 95 mol-%, bezogen auf die Gesamtmenge der bei der Herstellung des Polyesters (B1) eingesetzten Monomere, mindestens ein difunktionelles Monomer (b1) mit aliphatischen Gruppen mit 12 bis 70 Kohlenstoffatomen zwischen den funktionellen Gruppen eingesetzt wird,
und
(B2) zur Herstellung der Diesterderivate mindestens eine dihydroxyfunktionelle Komponente, welche eine aliphatische Gruppe mit 12 bis 70 Kohlenstoffatomen zwischen den Hydroxylgruppen aufweist,
eingesetzt wird.

12. Wässrige Zusammensetzung enthaltend mindestens ein Poly- und/oder Diesterderivat gemäß einem oder mehreren der Ansprüche 1 bis 10 sowie Wasser.

13. Wässrige Zusammensetzung nach Anspruch 12, **dadurch gekennzeichnet, dass** sie zusätzlich ein Neutralisierungsmittel ausgewählt aus der Gruppe bestehend aus Ammoniak, Aminen und/oder Aminoalkoholen enthalten.

14. Wässrige Zusammensetzung nach Anspruch 12 oder 13, **dadurch gekennzeichnet, dass** der Gehalt des mindestens einen Poly- und/oder Diesterderivats, bezogen auf die Gesamtkomposition, 20 bis 65 Gew.-% beträgt.

15. Verwendung von Poly- und Diesterderivaten gemäß einem der Ansprüche 1 bis 10 zur Herstellung flüssigkristalliner Phasen.

## Claims

1. Carboxy-functional polyester and diester derivatives preparable by ring-opening reaction of
(A) at least one anhydride of the formula (I) 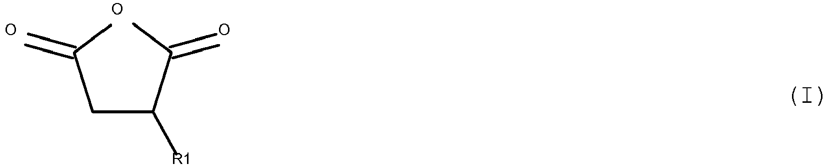 where R₁ = H, C₁ to C₄₈ alkyl, or C₂ to C₄₈ alkenyl
with
(B) at least one hydroxy-functional component, where
(B1) the polyester derivatives are prepared using at least one linear polyester of the two terminal hydroxyl groups, prepared using 7 to 95 mol%, based on the total amount of the monomers used in preparing the polyester (B1), of at least one difunctional monomer (b1) with aliphatic groups with 12 to 70 carbon atoms between the functional groups,
and
(B2) the diester derivatives are prepared using at least one dihydroxy-functional component which has an aliphatic group with 12 to 70 carbon atoms between the hydroxyl groups.

2. Carboxy-functional polyester and diester derivatives according to Claim 1, wherein for the formula (I) R₁ is H and/or C₆ to C₄₈ alkenyl.

3. Carboxy-functional polyester and diester derivatives according to Claim 1 or 2, wherein the aliphatic groups of the monomers (b1) and of component (B2) have 13 to 50 carbon atoms.

4. Carboxy-functional polyester and diester derivatives according to one or more of Claims 1 to 3, wherein the monomers (b1) are selected from the group consisting of fully hydrogenated bisphenols, dimeric aliphatic fatty alcohols, and dimeric aliphatic fatty acids.

5. Carboxy-functional polyester and diester derivatives according to one or more of Claims 1 to 4, wherein the polyester (B1) has a hydroxyl number of 80 to 200 mg KOH/g.

6. Carboxy-functional polyester and diester derivatives according to one or more of Claims 1 to 5, wherein component (B2) is selected from the group consisting of fully hydrogenated bisphenols and/or dimeric aliphatic fatty alcohols.

7. Carboxy-functional polyester and diester derivatives according to one or more of Claims 1 to 6, wherein the molar ratio of the anhydride groups of component (A) to the hydroxyl groups of component (B) in the ring-opening reaction is from 0.7 to less than 1.0.

8. Carboxy-functional polyester and diester derivatives according to one or more of Claims 1 to 7, having an acid number of between 50 and 250 mg KOH/g.

9. Carboxy-functional polyester and diester derivatives according to one or more of Claims 1 to 8, having an OH number of 2 to 30 mg KOH/g.

10. Carboxy-functional polyester and diester derivatives according to one or more of Claims 1 to 9, wherein the polyesters (B1) are prepared using 60 to 90 mol%, based on the total amount of the monomers used in preparing the polyester (B1), of at least one monomer (B1).

11. Process for preparing polyester and diester derivatives according to one or more of Claims 1 to 10, by reacting
(A) at least one anhydride of the formula (I) 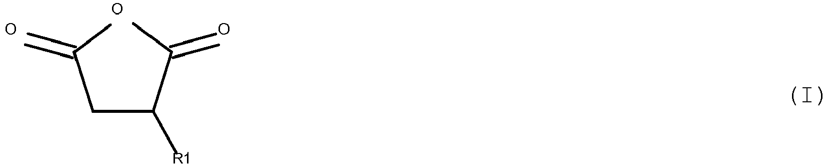 where R₁ = H, C₁ to C₄₈ alkyl, or C₂ to C₄₈ alkenyl
with
(B) at least one hydroxy-functional component, with opening of the anhydride ring, where
(B1) the polyester derivatives are prepared using at least one linear polyester of the two terminal hydroxyl groups, prepared using 7 to 95 mol%, based on the total amount of the monomers used in preparing the polyester (B1), of at least one difunctional monomer (b1) with aliphatic groups with 12 to 70 carbon atoms between the functional groups,
and
(B2) the diester derivatives are prepared using at least one dihydroxy-functional component which has an aliphatic group with 12 to 70 carbon atoms between the hydroxyl groups.

12. Aqueous composition comprising at least one polyester derivative and/or diester derivative according to one or more of Claims 1 to 10, and water.

13. Aqueous composition according to Claim 12, further comprising a neutralizing agent selected from the group consisting of ammonia, amines and/or amino alcohols.

14. Aqueous composition according to Claim 12 or 13, wherein the amount of the at least one polyester derivative and/or diester derivative, based on the overall constitution, is 20 to 65 wt%.

15. Use of polyester and diester derivatives according to any of Claims 1 to 10 for producing liquid-crystalline phases.

## Revendications

1. Dérivés de polyester et diester carboxy-fonctionnels pouvant être préparés par réaction d'ouverture de cycle de
(A) au moins un anhydride de formule (I) 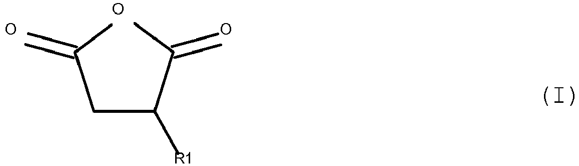 où R₁ = H, alkyle en C₁ à C₄₈, ou alcényle en C₂ à C₄₈
avec
(B) au moins un composant hydroxy-fonctionnel, où
(B1) les dérivés de polyester sont préparés en utilisant au moins un polyester linéaire des deux groupes hydroxyle, terminaux préparés en utilisant 7 à 95 % en moles, sur la base de la quantité totale des monomères utilisés dans la préparation du polyester (B1), d'au moins un monomère difonctionnel (b1) avec des groupes aliphatiques ayant 12 à 70 atomes de carbone entre les groupes fonctionnels,
et
(B2) les dérivés de diester sont préparés en utilisant au moins un composant dihydroxy-fonctionnel qui a un groupe aliphatique ayant 12 à 70 atomes de carbone entre les groupes hydroxyle.

2. Dérivés de polyester et diester carboxy-fonctionnels selon la revendication 1, dans lesquels, pour la formule (I) R₁ est H et/ou alcényle en C₆ à C₄₈.

3. Dérivés de polyester et diester carboxy-fonctionnels selon la revendication 1 ou 2, dans lesquels les groupes aliphatiques des monomères (b1) et du composant (B2) ont 13 à 50 atomes de carbone.

4. Dérivés de polyester et diester carboxy-fonctionnels selon une ou plusieurs des revendications 1 à 3, dans lesquels les monomères (b1) sont choisis dans le groupe constitué de bisphénols complètement hydrogénés, d'alcools gras aliphatiques dimères et d'acides gras aliphatiques dimères.

5. Dérivés de polyester et diester carboxy-fonctionnels selon une ou plusieurs des revendications 1 à 4, dans lesquels le polyester (B1) a un indice d'hydroxyle de 80 à 200 mg KOH/g.

6. Dérivés de polyester et diester carboxy-fonctionnels selon une ou plusieurs des revendications 1 à 5, dans lesquels le composant (B2) est choisi dans le groupe constitué de bisphénols complètement hydrogénés et/ou d'alcools gras aliphatiques dimères.

7. Dérivés de polyester et diester carboxy-fonctionnels selon une ou plusieurs des revendications 1 à 6, dans lesquels le rapport molaire des groupes anhydride du composant (A) aux groupes hydroxyle du composant (B) dans la réaction d'ouverture de cycle est de 0,7 à moins de 1,0.

8. Dérivés de polyester et diester carboxy-fonctionnels selon une ou plusieurs des revendications 1 à 7, ayant un indice d'acide compris entre 50 et 250 mg KOH/g.

9. Dérivés de polyester et diester carboxy-fonctionnels selon une ou plusieurs des revendications 1 à 8, ayant un indice OH de 2 à 30 mg KOH/g.

10. Dérivés de polyester et diester carboxy-fonctionnels selon une ou plusieurs des revendications 1 à 9, dans lesquels les polyesters (B1) sont préparés en utilisant 60 à 90 % en moles, sur la base de la quantité totale des monomères utilisés dans la préparation du polyester (B1), d'au moins un monomère (B1).

11. Procédé de préparation de polyester et de dérivés de diester selon une ou plusieurs des revendications 1 à 10, par réaction de
(A) au moins un anhydride de formule (I) 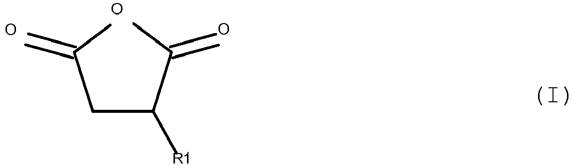 où R₁ = H, alkyle en C₁ à C₄₈, ou alcényle en C₂ à C₄₈
avec
(B) au moins un composant hydroxy-fonctionnel, avec ouverture du cycle anhydride, où
(B1) les dérivés de polyester sont préparés en utilisant au moins un polyester linéaire des deux groupes hydroxyle terminaux, préparé en utilisant 7 to 95 % en moles, sur la base de la quantité totale des monomères utilisés dans la préparation du polyester (B1), d'au moins un monomère difonctionnel (b1) avec des groupes aliphatiques ayant 12 à 70 atomes de carbone entre les groupes fonctionnels,
et
(B2) les dérivés de diester sont préparés en utilisant au moins un composant dihydroxy-fonctionnel qui comporte un groupe aliphatique ayant 12 à 70 atomes de carbone entre les groupes hydroxyle.

12. Composition aqueuse comprenant au moins un dérivé de polyester et/ou un dérivé de diester selon une ou plusieurs des revendications 1 à 10, et de l'eau.

13. Composition aqueuse selon la revendication 12, comprenant en outre un agent neutralisant choisi dans le groupe constitué de l'ammoniac, d'amines et/ou d'amino-alcools.

14. Composition aqueuse selon la revendication 12 ou 13, dans laquelle la quantité de l'au moins un dérivé de polyester et/ou dérivé de diester, sur la base de la constitution totale, est de 20 à 65 % en poids.

15. Utilisation de polyester et de dérivés de diester selon l'une quelconque des revendications 1 à 10 pour produire des phases cristallines liquides.
